# EUROPÄISCHE PATENTSCHRIFT

(11) **EP 4 216 840 B1**
(45) Veröffentlichungstag und Bekanntmachung des Hinweises auf die Patenterteilung: **10.06.2026**
(21) Anmeldenummer: 21783227.8
(22) Anmeldetag: 24.09.2021
(51) Int. Cl.: A61B 17/28, A61B 17/30, A61B 17/29

(54) **MEDIZINISCHES INSTRUMENT UND VERFAHREN ZUM HERSTELLEN EINES MEDIZINISCHEN INSTRUMENTS**
MEDICAL INSTRUMENT AND METHOD FOR PRODUCING A MEDICAL INSTRUMENT
INSTRUMENT MÉDICAL ET PROCÉDÉ PERMETTANT DE PRODUIRE UN INSTRUMENT MÉDICAL

(30) Priorität: 25.09.2020 DE 102020125070
(43) Veröffentlichungstag der Anmeldung: 02.08.2023
(73) Patentinhaber: Aesculap AG, 78532 Tuttlingen (DE)
(72) Erfinder: MORALES, Daniel, 78532 Nendingen (DE); FAITSCH, Dominic, 78579 Neuhausen (DE); WEISSER, Daniel, 78048 Villingen-Schwenningen (DE)
(74) Vertreter: Hoeger, Stellrecht & Partner Patentanwälte mbB
(86) Internationale Anmeldenummer: PCT/EP2021/076290
(87) Internationale Veröffentlichungsnummer: WO 2022/063944

(56) Entgegenhaltungen:
- DE-A1- 2 346 400
- US-A- 2 668 538
- US-A- 5 613 499
- US-A1- 2005 065 538
- US-A1- 2016 151 083

## Beschreibung

Die vorliegende Erfindung betrifft ein medizinisches Instrument mit mindestens einem Instrumentenarm, wobei an einem distalen Ende des Instrumentenarms eine Verzahnung mit mindestens zwei Zähnen ausgebildet ist, die quer vom distalen Ende abstehen, wobei jeder der mindestens zwei Zähne zwei voneinander weg weisende Zahnflanken aufweist.

Ferner betrifft die vorliegende Erfindung ein Verfahren zum Herstellen eines medizinischen Instruments, welches mindestens einen Instrumentenarm umfasst, wobei an einem distalen Ende des Instrumentenarms eine Verzahnung mit mindestens zwei Zähnen ausgebildet wird, die quer vom distalen Ende abstehen, wobei jeder der mindestens zwei Zähne zwei voneinander weg weisende Zahnflanken aufweist.

Medizinische Instrumente der eingangs beschriebenen Art sind insbesondere in Form von Pinzetten mit Verzahnungen an distalen Enden von Pinzettenarmen der Pinzette bekannt, wobei die Verzahnungen ein oder mehrere sogenannte Mauszähne umfassen, die in einer Schließstellung der Pinzette ineinandergreifen. Die Herstellung derartiger Instrumente ist mit einem hohen Aufwand verbunden. Die Verzahnungen werden üblicherweise von Hand gefertigt und erfordern ein hohes Geschick eines Chirurgiemechanikers, damit die Verzahnungen in der Schließstellung perfekt ineinandergreifen.

Aus der US 2016/0151083 A1 ist eine medizinische Greifvorrichtung bekannt. Ein asymmetrischer Shaver und Verfahren zum Herstellen desselben sind in der US 2005/0065538 A1 beschrieben. Aus der US 2,66,538 ist eine chirurgische Klemmvorrichtung bekannt. In der DE 23 46 400 A1 ist eine Pinzette beschrieben. Eine endoskopische Biopsiepinzette ist in der US 5,613,499 offenbart.

Es ist daher eine Aufgabe der vorliegenden Erfindung, ein medizinisches Instrument der eingangs beschriebenen Art und ein Verfahren zum Herstellen eines medizinischen Instruments der eingangs beschriebenen Art so zu verbessern, dass eine Herstellung des medizinischen Instruments vereinfacht wird.

Diese Aufgabe wird erfindungsgemäß durch ein medizinisches Instrument mit den Merkmalen des Anspruchs 1 gelöst.

Die vorgeschlagene Ausgestaltung der Verzahnung des mindestens einen Instrumentenarms ermöglicht insbesondere die Ausbildung der Zähne auf einfache Weise und insbesondere vollautomatisch, beispielsweise mit einer CNC-Maschine. Zum Ausbilden der ersten und zweiten Flächenbereiche werden lediglich Fräswerkzeuge benötigt. Insbesondere können die gegeneinander geneigten Flächenbereiche mit zwei unterschiedlichen Fräswerkzeugen ausgebildet werden. Beispielsweise können auch Rotationsachsen der Fräswerkzeuge beim Herstellen gegeneinander geneigt werden. Ferner ermöglichen es die gegeneinander geneigten Flächenbereiche an jeder Zahnflanke, dass das Instrument, wenn es zwei Instrumentenarme mit in einer Schließstellung ineinandergreifender Verzahnungen umfasst, die Verzahnungen nicht oder nur minimal von Hand nachgearbeitet werden müssen, dass sie vollständig und perfekt ineinandergreifen. Insbesondere durch das Ausbilden des ersten Flächenbereichs ist es möglich, die Instrumentenarme in der Schließstellung des Instruments vollständig aneinander anzulegen. Durch die besondere Ausgestaltung der Verzahnung ist es möglich, diese insbesondere vollständig maschinell auszubilden. So lässt sich eine Herstellungszeit verkürzen und mit der Herstellung einhergehende Kosten senken. Zudem ist kein besonderes handwerkliches Geschick erforderlich, um die Verzahnung auszubilden.

Günstig ist es, wenn die zwei Zahnflanken jedes der mindestens zwei Zähne spiegelsymmetrisch oder im Wesentlichen spiegelsymmetrisch zu einer Zahnspiegelebene ausgebildet sind und wenn eine Schnittlinie der mindestens zwei zweiten Flächenbereiche des jeweiligen Zahnes in der Zahnspiegelebene liegt.

Derart ausgebildete Zähne ermöglichen ein optimales Schließen des Instruments in der Schließstellung, also wenn Verzahnungen von zwei Instrumentenarmen ineinandergreifen.

Die Verzahnung lässt sich auf einfache Weise und mit einer hohen Symmetrie ausbilden, wenn eine Zahnspitze des Zahns auf der Schnittlinie der mindestens zwei zweiten Flächenbereiche des jeweiligen Zahnes liegt.

Günstig ist es, wenn ein distaler Endbereich des mindestens einen Instrumentenarms eine Längsrichtung definiert und wenn die Verzahnung bezogen auf die Längsrichtung quer abstehend ausgebildet ist. So lässt sich auf einfache Weise mit zwei derartigen Instrumentenarmen beispielsweise eine Pinzette ausbilden.

Vorteilhaft ist es, wenn der Instrumentenarm, insbesondere dessen distaler Endbereich, eine ebene Oberseite aufweist und wenn die Verzahnung mindestens teilweise über die Oberseite vorsteht. Instrumente mit derartigen Instrumentenarmen können insbesondere genutzt werden, um Gewebe präzise und feinfühlig zu präparieren.

Günstig ist es, wenn jeder erste Flächenbereich mit der Oberseite eine erste Oberseitenschnittlinie definiert und wenn jeder zweite Flächenbereich mit der Oberseite eine zweite Oberseitenschnittlinie definiert. Diese Ausgestaltung ermöglicht insbesondere ein optimales Ineinandergreifen von zusammenwirkenden Verzahnungen, die an zwei Instrumentenarmen ausgebildet sind.

Um insbesondere ein gutes Ineinandergreifen von Verzahnungen an zwei Instrumentenarmen zu ermöglichen, ist es vorteilhaft, wenn die erste Oberseitenschnittlinie und die zweite Oberseitenschnittlinie derselben Zahnflanke einen sich in proximaler Richtung öffnenden spitzen Winkel einschließen.

Auf einfache Weise lässt sich die Verzahnung mit einem Kegelfräser ausbilden, wenn die zweiten Oberseitenschnittlinien benachbarter Zähne der Verzahnung einen sich in distaler Richtung weisend geöffneten spitzen Winkel definieren.

Vorteilhaft ist es, wenn sich in einem Schnittpunkt der ersten Oberseitenschnittlinie und der zweiten Oberseitenschnittlinie der erste Flächenbereich und der zweite Flächenbereich einer Zahnflanke und die Oberseite berühren. Auf diese Weise kann ein perfektes Ineinandergreifen von Verzahnungen, die an zwei zusammenwirkenden Instrumentenarmen ausgebildet sind, erreicht werden.

Vorzugsweise verläuft die erste Oberseitenschnittlinie parallel oder im Wesentlichen parallel zur Längsrichtung. Dies hat insbesondere den Vorteil, dass Verzahnungen an zwei zusammenwirkenden Instrumentenarmen optimal ineinandergreifen können.

Günstig ist es, wenn jeder erste Flächenbereich mit einer Schrägfläche, welche relativ zur Oberseite um einen Schrägenwinkel geneigt ist, eine erste Schrägenflächenschnittlinie definiert und wenn jeder zweite Flächenbereich mit der Schrägfläche eine zweite Schrägflächenschnittlinie definiert. Auf diese Weise ist es insbesondere möglich, Verzahnungen an distalen Enden zusammenwirkender Instrumentenarme auch dann vollständig ineinandergreifen zu lassen, wenn die Längsachsen der Instrumentenarme nicht parallel zueinander ausgerichtet sind, sondern in distaler Richtung aufeinander zu laufen, also in proximaler Richtung einen Öffnungswinkel miteinander einschließen. Dies kann insbesondere dann der Fall sein, wenn die Instrumentenarme elastisch und etwas voneinander weg weisend ausgebaucht ausgebildet sind. Wenn keine parallele Ausrichtung der Instrumentenarme im Bereich ihrer distalen Enden sichergestellt werden kann, ermöglicht die relativ zur Oberseite um den Schrägenwinkel geneigte Schrägfläche dann wie beschrieben ein verbessertes Zusammenwirken der Verzahnungen der beiden Instrumentenarme. Die Ausgestaltung der Schrägfläche entspricht im Wesentlichen dem oben beschriebenen Flächenbereich, der von der ersten Oberseitenschnittlinie und der zweiten Oberseitenschnittlinie begrenzt wird. Dieser verläuft jedoch parallel zur Oberseite. Die Schrägfläche ist wie angegeben relativ zur Oberseite geneigt.

Vorteilhaft ist es, wenn der Schrägenwinkel einen Wert in einem Bereich von etwa 3° bis etwa 30° aufweist. Insbesondere kann der Schrägenwinkel einen Wert in einem Bereich von etwa 8° bis etwa 13° aufweisen. Weiter insbesondere kann ein Wert des Schrägenwinkels etwa 10° betragen. Den Schrägenwinkel in den angegebenen Bereichen vorzusehen hat insbesondere den Vorteil, dass Verzahnungen an distalen Enden nicht parallel verlaufender Instrumentenarme auch noch optimal ineinandergreifen können.

Günstig ist es, wenn sich die Schrägfläche und die Oberseite in einer Schrägflächenoberseitenschnittlinie schneiden, die quer, insbesondere senkrecht, zur Längsrichtung verläuft. Die Schrägflächenoberseitenschnittlinie bildet somit eine Begrenzungslinie der Schrägfläche in proximaler Richtung.

Vorteilhaft ist es, wenn die erste Schrägflächenschnittlinie und die zweite Schrägflächenschnittlinie derselben Zahnflanke einen sich in proximaler Richtung öffnenden spitzen Winkel einschließen. Diese Ausgestaltung ermöglicht insbesondere ein gutes Ineinandergreifen von Verzahnungen an zwei Instrumentenarmen.

Die Verzahnung lässt sich auf einfache Weise mit einem Kegelfräser ausbilden, wenn die zweiten Schrägflächenschnittlinien benachbarter Zähne der Verzahnung einen sich in distaler Richtung weisend geöffneten spitzen Winkel definieren.

Günstig ist es, wenn sich in einem Schnittpunkt der ersten Schrägflächenschnittlinie und der zweiten Schrägflächenschnittlinie der erste Flächenbereich und der zweite Flächenbereich einer Zahnflanke und die Schrägfläche berühren. So lässt sich insbesondere ein verbessertes Ineinandergreifen von Verzahnungen, die an zwei zusammenwirkenden Instrumentenarmen ausgebildet sind, erreichen.

Vorzugsweise sind die erste Schrägflächenschnittlinie und die Längsrichtung um den schrägen Winkel gegeneinander geneigt. So können insbesondere auch Verzahnungen an zwei zusammenwirkenden Instrumentenarmen besser ineinandergreifen, wenn die Instrumentenarme nicht parallel zueinander ausgerichtet sind, sondern einen in proximaler Richtung weisenden Öffnungswinkel einschließen.

Günstigerweise verlaufen die mindestens zwei ersten Flächenbereiche der beiden voneinander weg weisenden Zahnflanken parallel oder im Wesentlichen parallel zueinander. Dies ermöglicht es insbesondere, eine korrespondierende Verzahnung mit der Verzahnung in einer Schließstellung des Instruments in Eingriff zu bringen, und zwar derart, dass Oberseiten der Instrumentenarme, von denen die Verzahnungen vorstehend ausgebildet sind, in einer Schließstellung aneinander anliegen.

Auf einfache Weise lässt sich die Verzahnung ausbilden, wenn der mindestens eine erste Flächenbereich mit einem ersten Fräswerkzeug ausgebildet ist. Insbesondere kann er mit einem Zylinderfräser, der auch als Schaftfräser bezeichnet wird, ausgebildet sein. So lassen sich die ersten Flächenbereiche auf einfache Weise parallel zueinander und senkrecht zur Oberseite des Instrumentenarms ausbilden. Statt eines Zylinderfräsers können auch Sägeblätter zum Einsatz kommen, mit denen der mindestens eine erste Flächenbereich durch Sägen ausgebildet wird.

Um eine Zahl an Arbeitsschritten zu minimieren, ist es vorteilhaft, wenn ein Flächenabstand der ersten Flächenbereiche benachbarter Zähne einem Durchmesser des Zylinderfräsers entspricht.

Eine maschinelle Fertigung der Verzahnung kann auf einfache Weise insbesondere dadurch erreicht werden, dass der mindestens eine ebene zweite Flächenbereich mit einem zweiten Fräswerkzeug, insbesondere einem ersten Kegelfräser, ausgebildet ist. Insbesondere kann der mindestens eine ebene zweite Flächenbereich der Zahnflanke nach der Ausbildung der ersten Flächenbereiche der Verzahnung ausgebildet werden.

Ferner kann vorgesehen sein, dass jeder Zahn mindestens einen ebenen dritten Flächenbereich aufweist. Dieser kann insbesondere in proximaler Richtung weisend und gegenüber der Oberseite geneigt ausgebildet sein.

Die Herstellung des Instruments kann insbesondere auf einfache Weise realisiert werden, wenn der mindestens eine dritte Flächenbereich durch Kaltverformen oder durch Fräsen, insbesondere mit einer CNC-Maschine, ausgebildet ist. Eine Bearbeitung der Verzahnung durch Fräsen ist insbesondere nur dann erforderlich, wenn der dritte Flächenbereich nicht durch Kaltverformen in der gewünschten Weise ausgebildet werden kann. Durch das Fräsen kann eine durch eine Kaltumformung ausgebildete unebene Oberfläche auf einfache Weise eben ausgebildet werden.

Die Ausbildung des mindestens einen dritten Flächenbereichs lässt sich auf einfache Weise realisieren, wenn er mit einem dritten Fräswerkzeug ausgebildet ist. Insbesondere kann das dritte Fräswerkzeug in Form eines zweiten Kegelfräsers ausgebildet sein. Somit können in der beschriebenen Weise mit lediglich drei Fräsvorgängen eine Zahnflanke mit einem ersten und einem zweiten Flächenbereich sowie ein dritter Flächenbereich eines Zahnes maschinell ausgebildet werden.

Vorteilhaft ist es, wenn der mindestens eine dritte Flächenbereich gemeinsame Schnittlinien mit den mindestens zwei ersten Flächenbereichen und mit den mindestens zwei zweiten Flächenbereichen des jeweiligen Zahns aufweist. Auf diese Weise lässt sich insbesondere ein vollständig symmetrischer Zahn ausbilden.

Um Gewebe sicher und feinfühlig präparieren zu können, ist es günstig, wenn der mindestens eine dritte Flächenbereich mit der Oberseite einen Öffnungswinkel einschließt und wenn der Öffnungswinkel einen Wert von mindestens 90° aufweist.

Vorteilhafterweise weist der Öffnungswinkel einen Wert in einem Bereich von etwa 110° bis etwa 140° auf. Insbesondere kann er einen Wert von etwa 125° aufweisen. Mit derartigen Zähnen lässt sich Gewebe sicher fassen und handhaben.

Auf einfache Weise lässt sich der Öffnungswinkel mit einem gewünschten Wert ausbilden, wenn er einem halben Kegelwinkel des zweiten Kegelfräsers zuzüglich 90° entspricht. Der mindestens eine dritte Flächenbereich kann dann auf einfache Weise ausgebildet werden, indem ein Kegelfräser quer zur Längsrichtung an den Zähnen vorbeibewegt wird, wobei eine Längsachse des Kegelfräsers senkrecht zur Oberseite verläuft.

Ferner ist es vorteilhaft, wenn der dritte Flächenbereich eine dritte Flächenbereichebene definiert, wenn die dritte Flächenbereichebene und die Oberseite eine dritte Oberseitenschnittlinie definieren und wenn sich die dritte Oberseitenschnittlinie quer, insbesondere senkrecht, zur Längsrichtung erstreckt. So lassen sich insbesondere hakenförmige Zähne auf einfache Weise ausbilden.

Das Instrument lässt sich auf einfache Weise ausbilden, wenn der mindestens eine Instrumentenarm aus einem Instrumentenarmrohling ausgebildet ist. Es kann also insbesondere ein Instrumentenarmrohling bereitgestellt werden, welcher einen Vorsprung am distalen Ende aufweist, der zur Ausbildung der Verzahnung am Instrumentenarm wie beschrieben bearbeitet wird, insbesondere durch maschinelles Fräsen.

Günstig ist es, wenn der Instrumentenarmrohling einen sich in distaler Richtung erstreckenden Armabschnitt mit einem rechteckigen Querschnitt aufweist und wenn an einem distalen Armabschnittsende ein quer, insbesondere senkrecht, abstehender Zahnvorsprung ausgebildet ist und wenn die Verzahnung am Zahnvorsprung ausgebildet ist. Der Zahnvorsprung kann insbesondere eine Form oder Außenkontur aufweisen, die beispielsweise durch ausschließlich maschinelles Bearbeiten verändert werden kann, um die Verzahnung auszubilden. Der Zahnvorsprung ist insbesondere vorzugsweise nur so groß, dass von einer Außenkontur desselben nur minimal Material entfernt werden muss, um die Verzahnung zu erzeugen.

Vorteilhaft ist es, wenn der Instrumentenrohling durch Kaltverformen aus einem metallischen Werkstoff ausgebildet ist. Insbesondere kann er aus einem Instrumentenstahl ausgebildet sein. Ein solcher Instrumentenrohling kann einfach und kostengünstig bereitgestellt werden.

Vorzugsweise umfasst die Verzahnung maximal sechs Zähne. Dies ermöglicht es insbesondere, Instrumente mit einer ausreichenden Anzahl an Zähnen bereitzustellen, jedoch eine Breite des Instruments auf ein sinnvolles Maß zu begrenzen.

Günstigerweise definieren die zweiten Flächenbereiche benachbarter Zähne zwischen sich einen Keilwinkel. Diese Ausgestaltung ermöglicht es insbesondere, die zwei Flächenbereiche benachbarter Zähne mit einem Kegelfräser auszubilden.

Um eine Funktion der Verzahnung in gewünschter Weise sicherstellen zu können, ist es insbesondere vorteilhaft, wenn der Keilwinkel einen Wert in einem Bereich von etwa 15° bis etwa 45° aufweist. Beispielsweise kann er auch einen Wert von etwa 25° bis etwa 35° aufweisen. Insbesondere lassen sich derartige Keilwinkel mit Kegelfräsern auf einfache Weise erzeugen.

Die Handhabung sowie die Herstellung des Instruments werden deutlich vereinfacht, wenn die Verzahnung spiegelsymmetrisch zu einer sich in Längsrichtung erstreckenden Verzahnungsspiegelebene ausgebildet ist. Insbesondere vereinfacht dies eine Programmierung einer CNC-Maschine zur Ausbildung der Verzahnung.

Gemäß einer weiteren bevorzugten Ausführungsform der Erfindung kann vorgesehen sein, dass jeder zweite Flächenbereich eine zweite Flächenbereichebene definiert, dass die zweiten Flächenbereichebenen benachbarter Zähne eine gemeinsame Flächenbereichschnittlinie definieren und dass die Flächenbereichschnittlinie mit der Längsrichtung einen in distaler Richtung geöffneten Neigungswinkel einschließt. Eine solche Ausgestaltung kann auf einfache Weise mit einem Kegelfräser realisiert werden, dessen Längsachse senkrecht zur Flächenbereichschnittlinie bewegt wird.

Um ein gutes Schließergebnis beim Ineinandergreifen zweier Verzahnungen von zwei Instrumentenarmen zu ermöglichen, ist es vorteilhaft, wenn der Neigungswinkel einen Wert in einem Bereich von etwa 30° bis etwa 50° aufweist. Insbesondere kann der Neigungswinkel etwa 40° betragen.

Günstig ist es, wenn jede Zahnflanke einen Einschnitt des zweiten Flächenbereichs aufweist und wenn der Einschnitt einerseits vom ersten Flächenbereich und andererseits von der Oberseite begrenzt ist. Ein solcher Einschnitt stellt insbesondere sicher, dass zwei korrespondierend zueinander ausgebildete Verzahnungen, bei denen die Zahnflanken derartige Einschnitte aufweisen, optimal ineinandergreifen können. Insbesondere ist es so möglich, dass Oberseiten der Instrumentenarme in einer Schließstellung aneinander anliegen können.

Vorzugsweise ist eine in distaler Richtung weisende Endfläche der Verzahnung abgerundet. So kann das Instrument in Gewebe eingeführt werden, ohne dies zu verletzen.

Um insbesondre ein möglichst atraumatisches Instrument ausbilden zu können, ist es günstig, wenn sich die Endfläche von einer Unterseite des Instrumentenarms, die in einer Richtung entgegengesetzt zur Oberseite weist, bis zur Zahnspitze erstreckt. Dies ermöglicht es insbesondere, ein distales Ende des Instruments mit zwei Instrumentenarmen so auszubilden, dass praktisch keine scharfen Kanten verbleiben, wenn die Verzahnungen der beiden Instrumentenarme ineinandergreifen.

Auf einfache Weise lässt sich die Endfläche abrunden, wenn sie eine Zylinderfläche definiert und wenn eine Zylinderlängsachse der Zylinderfläche quer, insbesondere senkrecht, zur Längsrichtung und parallel zur Oberseite verläuft. So kann die Endfläche insbesondere die Form eines halben Zylinders aufweisen. Miteinander zusammenwirkende Verzahnungen mit entsprechend geformten Endflächen können dann ein halbzylindrisch geformtes distales Ende des Instruments in der Schließstellung definieren.

Die Gefahr von Verletzungen von Gewebe durch das medizinische Instrument kann auf einfache Weise insbesondere dadurch minimiert werden, dass die Endfläche einen Ausschnitt einer Kugeloberfläche definiert oder im Wesentlichen kugelig ausgebildet ist.

Um eine hinreichende Stabilität des Instruments, insbesondere im Bereich der Verzahnung, zu erreichen, ist es vorteilhaft, wenn ein Abstand der Zahnspitze von der Oberseite kleiner ist als eine Dicke des Instrumentenarms im Bereich seines distalen Endes und wenn die Dicke definiert ist durch einen Abstand der Oberseite von der Unterseite. Mit anderen Worten steht die Zahnspitze von der Oberseite nicht weiter vor als der Instrumentenarm im Bereich des distalen Endes dick ist.

Gemäß einer weiteren bevorzugten Ausführungsform der Erfindung kann vorgesehen sein, dass das Instrument zwei Instrumentenarme umfasst, dass an einem ersten Instrumentenarm der zwei Instrumentenarme eine erste Verzahnung ausgebildet ist, die in Richtung auf einen zweiten Instrumentenarm der zwei Instrumentenarme hin weist, dass am zweiten Instrumentenarm eine zweite Verzahnung ausgebildet ist, die in Richtung auf den ersten Instrumentenarm hin weist und dass in einer Schließstellung des Instruments, in welcher distale Enden der zwei Instrumentenarme einander maximal angenähert sind, die erste Verzahnung und die zweite Verzahnung ineinandergreifen. Ein solches Instrument kann beispielsweise in Form einer Pinzette ausgebildet werden, mit der Gewebe einfach und sicher gehandhabt werden kann. Insbesondere können die zwei Instrumentenarme des Instruments separat voneinander ausgebildet werden. Beispielsweise können die Instrumentenarme an proximalen Enden miteinander verbunden sein.

Vorzugsweise umfasst die zweite Verzahnung einen Zahn mehr als die erste Verzahnung. Auf diese Weise können die Instrumentenarme vollständig parallel zueinander verlaufen. Weist beispielsweise eine Verzahnung zwei Zähne auf und die andere drei Zähne, können die zwei Zähne in die Ausnehmungen zwischen den drei Zähnen in der Schließstellung eingreifen. Insbesondere können beide Verzahnungen spiegelsymmetrisch zu einer gemeinsamen Spiegelebene des Instruments ausgebildet sein. So kann insbesondere in einer Schließstellung ein nahezu perfekt geschlossenes distales Ende des Instruments ausgebildet werden.

Die Ausbildung des Instruments kann insbesondere dadurch weiter vereinfacht werden, dass die Zähne der ersten Verzahnung und der zweiten Verzahnung identisch oder im Wesentlichen identisch geformt sind.

Um insbesondere Gewebe einfach und sicher handhaben zu können, ist es vorteilhaft, wenn das Instrument in Form einer Pinzette ausgebildet ist.

Die eingangs gestellte Aufgabe wird ferner erfindungsgemäß durch ein Verfahren mit den Merkmalen des Anspruchs 7 gelöst.

Wie bereits oben erläutert, kann mit diesem Verfahren ein medizinisches Instrument, insbesondere eine Verzahnung desselben, vollständig oder im Wesentlichen vollständig maschinell ausgebildet werden. Derartige Instrumente lassen sich also wesentlich schneller als herkömmlich von Hand gefertigte Instrumente ausbilden. Dies ist zudem kostengünstiger und ermöglicht eine höhere Reproduzierbarkeit von Instrumenten mit stets gleichbleibender Qualität.

Vorteilhaft ist es, wenn die zwei Zahnflanken jedes der mindestens zwei Zähne spiegelsymmetrisch oder im Wesentlichen spiegelsymmetrisch zu einer Zahnspiegelebene ausgebildet werden und wenn eine Schnittlinie der mindestens zwei zweiten Flächenbereiche des jeweiligen Zahnes in der Zahnspiegelebene liegt. Werden zwei Verzahnungen mit derartigen Zähnen ausgebildet, ist insbesondere auf einfache Weise sicherstellbar, dass die Verzahnungen in einer Schließstellung optimal ineinandergreifen können.

Eine Herstellung des Instruments wird besonders einfach, wenn eine Zahnspitze des Zahns ausgebildet wird, die auf der Schnittlinie der mindestens zwei zweiten Flächenbereiche des jeweiligen Zahnes liegt. Ferner kann so insbesondere auch ein Ineinandergreifen korrespondierender Verzahnungen, die an zwei Instrumentenarmen ausgebildet sind, verbessert werden.

Günstig ist es, wenn ein distaler Endbereich des mindestens einen Instrumentenarms eine Längsrichtung definiert und wenn die Verzahnung bezogen auf die Längsrichtung quer abstehend ausgebildet wird. Mit einer derartig ausgerichteten Verzahnung kann insbesondere Gewebe optimal gefasst und manipuliert werden.

Vorteilhaft ist es, wenn der Instrumentenarm, insbesondere dessen distaler Endbereich, eine ebene Oberseite aufweist und wenn die Verzahnung mindestens teilweise über die Oberseite vorstehend ausgebildet wird. Die Verzahnung kann so hakenartig genutzt werden, um Gewebe handzuhaben, insbesondere es zu präparieren.

Günstig ist es, wenn jeder erste Flächenbereich derart ausgebildet wird, dass er mit der Oberseite eine erste Oberseitenschnittlinie definiert, und wenn jeder zweite Flächenbereich derart ausgebildet wird, dass er mit der Oberseite eine zweite Oberseitenschnittlinie definiert. Insbesondere können so auf einfache Weise gegeneinander geneigte erste und zweite Flächenbereiche zur Ausbildung einer Zahnflanke des Zahns realisiert werden. Auch kann insbesondere ein Ineinandergreifen korrespondierender Verzahnungen mit derart geformten Flächenbereichen verbessert werden.

Vorzugsweise wird jede Zahnflanke derart ausgebildet, dass die erste Oberseitenschnittlinie und die zweite Oberseitenschnittlinie einen sich in proximaler Richtung öffnenden spitzen Winkel einschließen. So kann insbesondere ein Einschnitt oder Rücksprung an jeder Zahnflanke ausgebildet werden, der teilweise durch einen ersten Flächenbereich und teilweise durch die Oberseite des Instrumentenarms im Bereich von dessen distalem Ende begrenzt wird.

Zueinander korrespondierend ausgebildete Verzahnungen können auf einfache Weise ineinander eingreifbar ausgebildet werden, wenn die Verzahnung derart ausgebildet wird, dass die zweiten Oberseitenschnittlinien benachbarter Zähne einen sich in distaler Richtung weisend geöffneten spitzen Winkel definieren. Insbesondere kann sich dieser Winkel ergeben aufgrund eines kegelförmigen Fräswerkzeugs, welches eingesetzt wird, um die zweiten Flächenbereiche auszubilden.

Günstig ist es, wenn die Verzahnung derart ausgebildet wird, dass sich in einem Schnittpunkt der ersten Oberseitenschnittlinie und der zweiten Oberseitenschnittlinie der erste Flächenbereich und der zweite Flächenbereich einer Zahnflanke und die Oberseite berühren. In dem Schnittpunkt grenzen also nicht nur die ersten und zweiten Flächenbereiche aneinander, sondern auch die Oberseite.

Vorteilhafterweise werden die Zähne derart ausgebildet, dass die erste Oberseitenschnittlinie parallel oder im Wesentlichen parallel zur Längsrichtung verläuft. Dies ermöglicht es insbesondere, korrespondierende Verzahnungen parallel zur Längsrichtung ineinander eingreifen zu lassen.

Günstig ist es, wenn jeder erste Flächenbereich derart ausgebildet wird, dass er mit einer Schrägfläche, welche relativ zur Oberseite um einen Schrägenwinkel geneigt ist, eine erste Schrägflächenschnittlinie definiert und wenn jeder zweite Flächenbereich derart ausgebildet wird, dass er mit der Schrägfläche eine zweite Schrägflächenschnittlinie definiert. Insbesondere können so auf einfache Weise gegeneinander geneigte erste und zweite Flächenbereiche zur Ausbildung einer Zahnflanke des Zahns realisiert werden. Auch kann insbesondere ein Ineinandergreifen korrespondierender Verzahnungen mit derart geformten Flächenbereichen verbessert werden, da es die relativ zur Oberseite geneigten Schrägflächen ermöglichen, dass Verzahnungen zusammenwirkender distaler Enden von zwei Instrumentenarmen eines medizinischen Instruments auch dann ineinandergreifen können, wenn die Instrumentenarme im Bereich ihrer distalen Enden nicht parallel zueinander verlaufen, sondern in distaler Richtung aufeinander zu laufen und so einen in proximaler Richtung weisenden Öffnungswinkel definieren.

Vorteilhaft ist es, wenn der Schrägenwinkel mit einem Wert in einem Bereich von etwa 3° bis etwa 30° ausgebildet wird. Insbesondere kann der Schrägenwinkel mit einem Wert in einem Bereich von etwa 8° bis etwa 13° ausgebildet werden. Weiter insbesondere kann der Schrägenwinkel einen Wert von etwa 10° aufweisen. Einen Schrägenwinkel in den genannten Bereichen vorzusehen ermöglicht es insbesondere, distale Enden zweier zusammenwirkender Instrumentenarme um den doppelten Schrägenwinkel gegeneinander zu neigen, wobei dann immer noch ein gutes Ineinandergreifen der an den distalen Enden der Instrumentenarme ausgebildeten Verzahnungen ermöglicht wird.

Günstig ist es, wenn jeder erste Flächenbereich derart ausgebildet wird, dass sich die Schrägfläche und die Oberseite in einer Schrägflächenoberseitenschnittlinie schneiden, die quer, insbesondere senkrecht, zur Längsrichtung verläuft. Die Schrägflächenoberseitenschnittlinie begrenzt also die Schrägfläche in proximaler Richtung.

Vorteilhaft ist es, wenn jede Zahnflanke derart ausgebildet wird, dass die erste Schrägflächenschnittlinie und die zweite Schrägflächenschnittlinie derselben Zahnflanke einen sich in proximaler Richtung öffnenden spitzen Winkel einschließen. Dies ermöglicht es insbesondere, einen Einschnitt oder Rücksprung an jeder Zahnflanke auszubilden, der teilweise durch einen ersten Flächenbereich und teilweise durch die Schrägfläche im Bereich von deren distalem Ende begrenzt wird.

Günstig ist es, wenn die Verzahnung derart ausgebildet wird, dass die zweiten Schrägflächenschnittlinien benachbarter Zähne der Verzahnung einen in distaler Richtung weisend geöffneten spitzen Winkel definieren. So können insbesondere korrespondierend zueinander ausgebildete Verzahnungen auf einfache Weise ineinander eingreifbar ausgebildet werden. Insbesondere kann sich der definierte spitze Winkel aufgrund eines kegelförmigen Fräswerkzeugs ergeben, welches eingesetzt wird, um die zweiten Flächenbereiche auszubilden.

Vorteilhaft ist es, wenn die Verzahnung derart ausgebildet wird, dass sich in einem Schnittpunkt der ersten Schrägflächenschnittlinie und der zweiten Schrägflächenschnittlinie der erste Flächenbereich und der zweite Flächenbereich einer Zahnflanke und die Schrägfläche berühren. In dem Schnittpunkt grenzen also nicht nur die ersten und zweiten Flächenbereiche aneinander, sondern auch die Schrägfläche.

Um ein optimales Ineinandergreifen von Verzahnungen zusammenwirkender Instrumentenarme zu ermöglichen, ist es vorteilhaft, wenn Zähne derart ausgebildet werden, dass die erste Schrägflächenschnittlinie und die Längsrichtung um den schrägen Winkel gegeneinander geneigt sind.

Gemäß einer weiteren bevorzugten Ausführungsform der Erfindung kann vorgesehen sein, dass die Zähne derart ausgebildet werden, dass die mindestens zwei ersten Flächenbereiche der beiden voneinander weg weisenden Zahnflanken parallel oder im Wesentlichen parallel zueinander verlaufen. Dies ermöglicht es insbesondere, dass bei korrespondierenden Verzahnungen die ersten Flächenbereiche der einen Verzahnung und die ersten Flächenbereiche der anderen Verzahnung parallel zueinander ausgerichtet sind, wenn die Verzahnungen ineinandergreifen. So kann ein Ineinandergreifen der Verzahnungen verbessert werden.

Günstig ist es, wenn der mindestens eine erste Flächenbereich mit einem ersten Fräswerkzeug ausgebildet wird. Insbesondere kann das erste Fräswerkzeug in Form eines Zylinderfräsers ausgebildet sein. Dabei ist es günstig, wenn eine Längsachse des Zylinderfräsers beim Fräsen sowohl senkrecht zur Oberseite als auch senkrecht zur Längsrichtung orientiert ist. So kann in einem ersten Schritt an einem Zahnvorsprung an einem Instrumentenarmrohling ein sich in distaler Richtung erstreckender Zahngrund mit dem Zylinderfräser ausgebildet werden. Statt des Zylinderfräsers kann auch ein Sägeblatt eingesetzt werden, um den mindestens eine ersten Flächenbereich auszubilden. Um alternativ insbesondere ein Schrägfläche ausbilden zu können, ist es günstig, wenn eine Längsachse des Zylinderfräsers beim Fräsen mit der Längsrichtung eine Fräsebene definiert, welche senkrecht zur Oberseite verläuft, und in distaler Richtung um einen Fräserlängsachsenwinkel geneigt ist. Insbesondere entspricht der Fräserlängsachsenwinkel dem Schrägenwinkel. So kann auf einfache Weise mit einem Zylinderfräser, dessen Längsachse nicht parallel zur Oberseite des Instrumentenarms verläuft, eine Schrägfläche ausgebildet werden.

Um eine Anzahl an Fräsvorgängen zu minimieren, ist es vorteilhaft, wenn die Verzahnung derart ausgebildet wird, dass ein Flächenabstand der ersten Flächenbereiche benachbarter Zähne einem Durchmesser des Zylinderfräsers entspricht.

Gemäß einer weiteren bevorzugten Ausführungsform der Erfindung kann vorgesehen sein, dass der mindestens eine ebene zweite Flächenbereich mit einem zweiten Fräswerkzeug ausgebildet wird. Insbesondere kann das zweite Fräswerkzeug in Form eines ersten Kegelfräsers ausgebildet sein. Ferner kann es vorteilhaft sein, wenn eine Längsachse des ersten Kegelfräsers beim Fräsen relativ zur Oberseite um einen in distaler Richtung geöffneten Neigungswinkel geneigt ist und mit der Längsrichtung eine zur Oberseite senkrecht verlaufende Ebene definiert. Die Längsachse des ersten Kegelfräsers wird also in der beschriebenen Ebene bewegt, und zwar nicht in Längsrichtung, sondern schräg relativ zu dieser. So lassen sich die zweiten Flächenbereiche benachbarter Zähne beispielsweise in einem einzigen Bearbeitungsschritt ausbilden.

Günstigerweise wird jeder Zahn mit mindestens einem ebenen dritten Flächenbereich ausgebildet wird. Der dritte Flächenbereich kann insbesondere eine Rückhaltefläche zum Rückhalten von Gewebe definieren. Der dritte Flächenbereich kann insbesondere mindestens teilweise in proximaler Richtung weisend ausgebildet werden.

Auf einfache Weise lässt sich der dritte Flächenbereich ausbilden, wenn er durch Kaltverformen oder durch Fräsen ausgebildet wird. Insbesondere kann er mit einer CNC-Maschine maschinell durch Fräsen ausgebildet werden. Eine kaltverformte Ausbildung des dritten Flächenbereichs kann insbesondere realisiert werden durch Bereitstellen eines Instrumentenarmrohlings, der bereits den dritten Flächenbereich aufweist und durch Kaltverformen ausgebildet wird.

Günstig ist es, wenn der mindestens eine dritte Flächenbereich mit einem dritten Fräswerkzeug ausgebildet wird. Insbesondere kann es sich dabei um einen zweiten Kegelfräser handeln. Insbesondere kann eine Längsachse des zweiten Kegelfräsers beim Fräsen sowohl senkrecht zur Oberseite als auch senkrecht zur Längsrichtung orientiert sein. Ein derart ausgerichteter zweiter Kegelfräser kann insbesondere quer zur Längsrichtung bewegt werden, um den mindestens einen dritten Flächenbereich an allen Zähnen der Verzahnung auszubilden.

Vorteilhaft ist es, wenn der Zahn derart ausgebildet wird, dass der mindestens eine dritte Flächenbereich gemeinsame Schnittlinien mit den mindestens zwei ersten Flächenbereichen und mit den mindestens zwei zweiten Flächenbereichen des jeweiligen Zahns aufweist. So kann insbesondere durch Ausbildung der ersten Flächenbereiche an jedem Zahn beziehungsweise an jeder Zahnflanke ein Rücksprung oder Einschnitt ausgebildet werden, der durch die Oberseite des Instrumentenarms und den ersten Flächenbereich begrenzt wird.

Günstig ist es, wenn der mindestens eine dritte Flächenbereich mit der Oberseite einen Öffnungswinkel einschließt und wenn der Öffnungswinkel einen Wert von mindestens 90° aufweist. Vorzugsweise handelt es sich beim Öffnungswinkel um einen stumpfen Winkel. Dies ermöglicht es insbesondere, dass Gewebe vom mindestens einen dritten Flächenbereich des Zahns in distaler Richtung abgleiten kann, wenn der Instrumentenarm mit der Verzahnung in proximaler Richtung bewegt wird.

Vorzugsweise wird der Öffnungswinkel mit einem Wert in einem Bereich von etwa 110° bis etwa 140° ausgebildet. Insbesondere kann der Öffnungswinkel einen Wert von etwa 125° aufweisen. Derartige Öffnungswinkel lassen sich insbesondere mit Kegelfräsern mit entsprechenden Kegelwinkeln auf einfache Weise ausbilden.

Auf einfache Weise lässt sich die Verzahnung realisieren, wenn der Öffnungswinkel durch einen halben Kegelwinkel des zweiten Kegelfräsers zuzüglich 90° vorgegeben wird.

Ferner kann es vorteilhaft sein, wenn der Zahn derart ausgebildet wird, dass der dritte Flächenbereich eine dritte Flächenbereichebene definiert, dass die dritte Flächenbereichebene und die Oberseite eine dritte Oberseitenschnittlinie definieren und dass sich die dritte Oberseitenschnittlinie quer, insbesondere senkrecht, zur Längsrichtung erstreckt. Ein derartiger Zahn lässt sich auf einfache Weise ausbilden, indem ein Kegelfräser mit seiner Längsachse senkrecht zur Oberseite in einer Richtung senkrecht zur Längsrichtung bewegt wird. So können insbesondere alle dritten Flächenbereiche der Verzahnung in einem Arbeitsgang ausgebildet werden.

Die Herstellung des Instruments kann insbesondere weiter vereinfacht werden, wenn der mindestens eine Instrumentenarm aus einem Instrumentenarmrohling ausgebildet wird. Beispielsweise kann der Instrumentenarmrohling durch Kaltverformen ausgebildet werden. Insbesondere kann er zum Herstellen des Instruments als Halbzeug bereitgestellt werden.

Günstig ist es, wenn der Instrumentenarmrohling mit einem sich in distaler Richtung erstreckenden Armabschnitt mit einem rechteckigen Querschnitt und mit einem an einem distalen Armabschnittsende quer, insbesondere senkrecht, abstehenden Zahnvorsprung ausgebildet wird und wenn die Verzahnung am Zahnvorsprung ausgebildet wird. Der Zahnvorsprung kann insbesondere als Rohform für einen Teil der Verzahnung genutzt werden. Die Verzahnung kann also insbesondere ausschließlich durch spanende Bearbeitung des Instrumentenarmrohlings ausgebildet werden.

Auf günstige Weise lässt sich ein Instrumentenarmrohling ausbilden, wenn er durch Kaltverformen aus einem metallischen Werkstoff ausgebildet wird. Beispielsweise kann es sich bei dem metallischen Werkstoff um einen Instrumentenstahl handeln. So kann eine ausreichende Stabilität des Instruments erreicht werden.

Vorzugsweise wird die Verzahnung mit maximal sechs Zähnen ausgebildet. Beispielsweise kann die Verzahnung auch drei, vier oder fünf Zähne aufweisen. Derartige Verzahnungen lassen sich einfach herstellen. Zudem kann so auch eine Breite eines distalen Endes des Instruments auf eine handhabbare Größe minimiert werden.

Günstig ist es, wenn die Verzahnung derart ausgebildet wird, dass die zweiten Flächenbereiche benachbarter Zähne zwischen sich einen Keilwinkel definieren. Eine solche Verzahnung lässt sich auf einfache Weise insbesondere dadurch ausbilden, dass zur Ausbildung der zweiten Flächenbereiche ein Kegelfräser zum Bearbeiten des Instrumentenarmrohlings genutzt wird.

Vorteilhaft ist es, wenn der Keilwinkel durch den ersten Kegelfräser vorgegeben wird und einen Wert in einem Bereich von etwa 15° bis etwa 45° aufweist. Insbesondere kann der Keilwinkel einen Wert von etwa 25° bis etwa 35° aufweisen. Derartige Keilwinkel können mit einem Kegelfräser einfach und sicher ausgebildet werden. Auch kann so ein Ineinandergreifen korrespondierender Verzahnungen auf einfache Weise sichergestellt werden.

Das Herstellen der Verzahnung lässt sich insbesondere dadurch vereinfachen, dass sie spiegelsymmetrisch zu einer sich in Längsrichtung erstreckenden Verzahnungsspiegelebene ausgebildet wird.

Gemäß einer weiteren bevorzugten Ausführungsform der Erfindung kann vorgesehen sein, dass die Verzahnung derart ausgebildet wird, dass jeder zweite Flächenbereich eine zweite Flächenbereichebene definiert, dass die zweiten Flächenbereichebenen benachbarter Zähne eine gemeinsame Flächenbereichschnittlinie definieren und dass die Flächenbereichschnittlinie mit der Längsrichtung einen in distaler Richtung geöffneten Neigungswinkel einschließt. Eine solche Verzahnung lässt sich auf einfache Weise ausbilden durch einen Kegelfräser, dessen Längsachse senkrecht zur Flächenbereichschnittlinie ausgerichtet und parallel zu dieser bewegt wird.

Günstig ist es, wenn der Neigungswinkel durch eine Orientierung der Längsachse des zweiten Kegelfräsers beim Fräsen relativ zur Oberseite vorgegeben wird und wenn die Längsachse senkrecht zur Flächenbereichschnittlinie ausgerichtet ist und mit der Längsrichtung eine zur Oberseite senkrecht verlaufende Ebene definiert. Der zweite Kegelfräser kann dann parallel zur Flächenbereichschnittlinie bewegt werden, um die zweiten Flächenbereiche benachbarter Zähne in einem einzigen Arbeitsschritt auszubilden. Insbesondere kann der Neigungswinkel einen Wert in einem Bereich von etwa 30° bis etwa 50° aufweisen. Beispielsweise kann er etwa 40° betragen. So kann insbesondere ein optimales Ineinandergreifen korrespondierender Verzahnungen, die an zwei zusammenwirkenden Instrumentenarmen ausgebildet sind, erreicht werden.

Günstigerweise werden die Zähne derart ausgebildet, dass jede Zahnflanke einen Einschnitt des zweiten Flächenbereichs aufweist und dass der Einschnitt einerseits vom ersten Flächenbereich und andererseits von der Oberseite begrenzt ist. Dieser Einschnitt, auch als Rücksprung bezeichnet, an jeder Zahnflanke ermöglicht es insbesondere, dass korrespondierend ausgebildete Verzahnungen so weit ineinandergreifen können, dass Oberseiten der Instrumentenarme, an denen die Verzahnungen ausgebildet sind, direkt miteinander in Anlage gebracht werden können.

Um ein atraumatisches Instrumentenende realisieren können, ist es vorteilhaft, wenn eine in distaler Richtung weisende Endfläche der Verzahnung abgerundet wird.

Vorzugsweise wird die Endfläche derart ausgebildet, dass sie sich von einer Unterseite des Instrumentenarms, die in einer Richtung entgegengesetzt zur Oberseite weist, bis zur Zahnspitze erstreckt. So kann insbesondere das distale Ende des Instruments vollständig abgerundet werden, auch wenn zwei zueinander korrespondierende Verzahnungen ineinandergreifen.

Vorteilhaft ist es, wenn die Endfläche derart ausgebildet wird, dass sie eine Zylinderfläche definiert und dass eine Zylinderlängsachse der Zylinderfläche quer, insbesondere senkrecht, zur Längsrichtung und parallel zur Oberseite verläuft. Insbesondere kann die Zylinderlängsachse in einer von der Oberseite definierten Ebene liegen. So kann insbesondere ein halbzylindrisches distales Ende des Instruments realisiert werden, wenn zwei zueinander korrespondierende Verzahnungen an zwei zusammenwirkenden Instrumentenarmen ausgebildet werden.

Des Weiteren kann es günstig sein, wenn die Endfläche derart ausgebildet wird, dass sie einen Ausschnitt einer Kugeloberfläche definiert oder im Wesentlichen kugelig ausgebildet ist. Derart ausgebildete Endflächen von Instrumentenarmen können insbesondere dazu beitragen, dass eine Gefahr von Gewebeverletzungen mit den distalen Enden der Instrumentenarme verringert wird.

Günstig ist es, wenn die Zähne derart ausgebildet werden, dass ein Abstand der Zahnspitze von der Oberseite kleiner ist als eine Dicke des Instrumentenarms im Bereich seines distalen Endes und dass die Dicke definiert ist durch einen Abstand der Oberseite von der Unterseite. Auf diese Weise kann eine für einen Einsatz des Instruments erforderliche Stabilität sichergestellt werden.

Gemäß einer weiteren bevorzugten Ausführungsform der Erfindung kann vorgesehen sein, dass das Instrument mit zwei Instrumentenarmen ausgebildet wird derart, dass an einem ersten Instrumentenarm der zwei Instrumentenarme eine erste Verzahnung ausgebildet wird, die in Richtung auf einen zweiten Instrumentenarm der zwei Instrumentenarme hin weist, dass am zweiten Instrumentenarm eine zweite Verzahnung ausgebildet wird, die in Richtung auf den ersten Instrumentenarm hin weist und dass in einer Schließstellung des Instruments, in welcher distale Enden der zwei Instrumentenarme einander maximal angenähert sind, die erste Verzahnung und die zweite Verzahnung ineinandergreifen. Auf diese Weise ist es insbesondere möglich, eine Pinzette mit korrespondierenden Verzahnungen an ihren distalen Arbeitsarmenden einfach und schnell auszubilden.

Um insbesondere ein symmetrisches Instrument ausbilden zu können, ist es günstig, wenn die zweite Verzahnung mit einem Zahn mehr als die erste Verzahnung ausgebildet wird. Beispielsweise kann eine Verzahnung drei Zähne aufweisen, die andere Verzahnung vier Zähne. Die drei Zähne der einen Verzahnung können dann in die drei Lücken der vier Zähnen umfassenden Verzahnung in der Schließstellung eingreifen.

Auf einfache Weise lässt sich das Instrument ausbilden, wenn die Zähne der ersten Verzahnung und der zweiten Verzahnung identisch oder im Wesentlichen identisch geformt werden.

Vorzugsweise wird das Instrument in Form einer Pinzette ausgebildet. Mit einer solchen Pinzette lässt sich Körpergewebe von Menschen oder Tieren einfach und sicher präparieren und handhaben.

Die nachfolgende Beschreibung bevorzugter Ausführungsformen der Erfindung dient im Zusammenhang mit den Zeichnungen der näheren Erläuterung. Es zeigen:
- Figur 1:: eine schematische Seitenansicht eines Ausführungsbeispiels eines medizinischen Instruments;
- Figur 2:: eine schematische perspektivische Ansicht eines distalen Endbereichs eines Instrumentenarmrohlings vor einem ersten Bearbeitungsschritt;
- Figur 3:: eine Ansicht ähnlich Figur 2 nach dem ersten Bearbeitungsschritt;
- Figur 4:: eine schematische perspektivische Ansicht des distalen Endbereichs des Instrumentenarmrohlings vor dem Durchführen eines zweiten Bearbeitungsschritts;
- Figur 5:: eine schematische perspektivische Ansicht des distalen Endbereichs des Instrumentenarmrohlings nach Durchführen eines dritten Bearbeitungsschritts;
- Figur 6:: eine Seitenansicht des bearbeiteten distalen Endbereichs des Instrumentenarmrohlings aus Figur 5;
- Figur 7:: eine Ansicht der Anordnung aus Figur 5 in Richtung des Pfeils A;
- Figur 8:: eine Ansicht der Anordnung aus Figur 7 in Richtung des Pfeils B;
- Figur 9:: eine Schnittansicht längs Linie 9-9 in Figur 7;
- Figur 10:: eine Ansicht der Anordnung aus Figur 9 in Richtung des Pfeils C;
- Figur 11:: eine perspektivische Ansicht des distalen Endbereichs des Instrumentenarms nach einem weiteren Bearbeitungsschritt;
- Figur 12:: eine Seitenansicht der Anordnung aus Figur 11;
- Figur 13:: eine Ansicht der Anordnung aus Figur 12 in Richtung des Pfeils D;
- Figur 14:: eine schematische perspektivische Ansicht eines distalen Endbereichs des Instruments aus Figur 1 in einer Schließstellung;
- Figur 15:: eine schematische perspektivische Ansicht des distalen Endbereichs des Instruments aus Figur 1 in geöffneter Stellung;
- Figur 16:: eine schematische perspektivische Ansicht eines weiteren Ausführungsbeispiels eines distalen Endbereichs eines Instrumentenarmrohlings mit drei Zähnen während beziehungsweise nach einem ersten Bearbeitungsschritt;
- Figur 17:: eine Schnittansicht längs Linie 17 - 17 in Figur 16;
- Figur 18:: eine schematische perspektivische Ansicht des distalen Endbereichs des Instrumentenarmrohlings aus den Figuren 16 und 17 nach dem Durchführen des in Figur 5 schematisch dargestellten Bearbeitungsschritts und dem Abrunden der distalen Endfläche;
- Figur 19:: eine Schnittansicht längs Linie 19 - 19 in Figur 18;
- Figur 20:: eine schematische perspektivische Ansicht des distalen Endbereichs eines weiteren Ausführungsbeispiels eines Instrumentenkörperteils ähnlich dem in Figur 18, jedoch mit nur zwei Zähnen; und
- Figur 21:: eine teilweise durchbrochene Schnittansicht ähnlich Figur 19 von distalen Enden zweier gegeneinander geneigter Instrumentenarme.

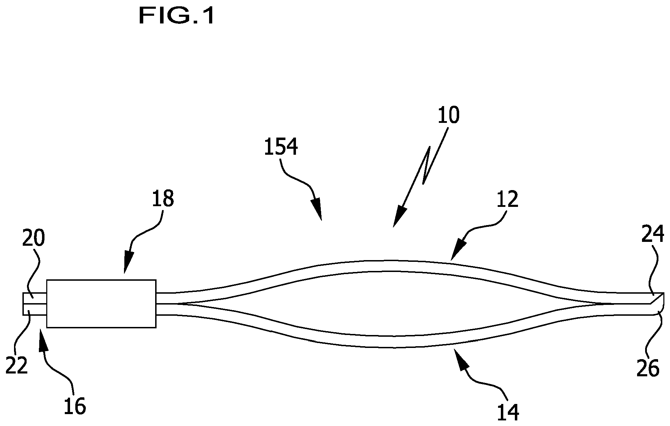

Ein Ausführungsbeispiel eines medizinischen Instruments ist schematisch in Figur 1 in einer Schließstellung dargestellt. Das Instrument 10 umfasst einen ersten Instrumentenarm 12 und einen zweiten Instrumentenarm 14.

Die Instrumentenarme 12 und 14 sind in einem proximalen Endbereich 16 mittels eines hülsenförmigen Verbindungselements 18 miteinander verbunden. Das Verbindungselement 18 umgreift proximale Endabschnitte 20 und 22 der beiden Instrumentenarme 12 und 14.

Die Instrumentenarme 12 und 14 erstrecken sich vom Verbindungselement 18 bis zu distalen Enden 24 beziehungsweise 26.

Die distalen Enden 24 und 26 sind in Figur 1 schematisch in einer maximal angenäherten Stellung dargestellt. Diese Schließstellung nimmt das Instrument 10 ein, wenn die Instrumentenarme 12 und 14 aufeinander zu bewegt sind, bis die distalen Enden 24 und 26 aneinander anliegen.

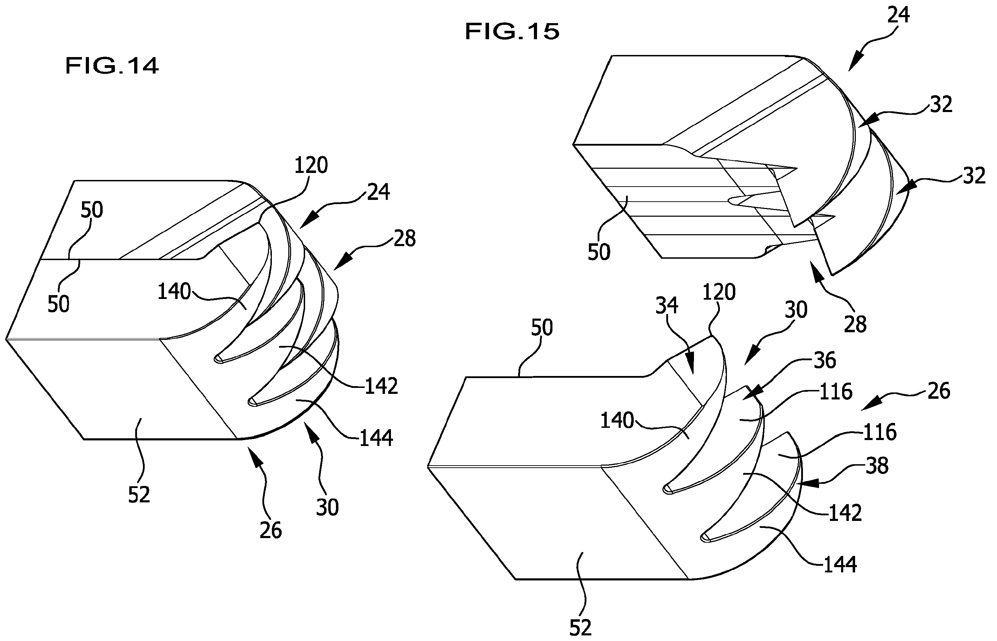

Die distalen Enden 24 und 26 sind schematisch in den Figuren 14 und 15 dargestellt. Am distalen Ende 24 ist eine erste Verzahnung 28 ausgebildet, am distalen Ende 26 eine zweite Verzahnung 30. In Figur 15 sind die distalen Enden 24 und 26 in einer voneinander entfernten oder geöffneten Stellung dargestellt. Figur 14 zeigt die distalen Enden 24 und 26 des Instruments 10 in einer maximal angenäherten Stellung, auch als Schließstellung bezeichnet, in welcher die Verzahnungen 28 und 30 ineinandergreifen.

Die erste Verzahnung 28 umfasst zwei identisch ausgebildete Zähne 32.

Die zweite Verzahnung 30 umfasst Zähne 34, 36 und 38. Der Zahn 36 ist identisch zu den Zähnen 32 ausgebildet. Die Zähne 34 und 38 sind spiegelsymmetrisch zueinander ausgebildet bezüglich einer Verzahnungsspiegelebene 40. Die Verzahnungsspiegelebene 40 definiert zudem eine Zahnspiegelebene 42, bezüglich derer der Zahn 36, der zwischen den Zähnen 34 und 38 angeordnet ist, ausgebildet ist.

In der Schließstellung greifen die Zähne 32 jeweils in eine Ausnehmung einerseits zwischen den Zähnen 34 und 36 und andererseits zwischen den Zähnen 36 und 38 ein.

Die beiden Verzahnungen 28 und 30 sind bei dem in Figur 1 dargestellten Ausführungsbeispiel des Instruments 10 vollständig maschinell ausgebildet.

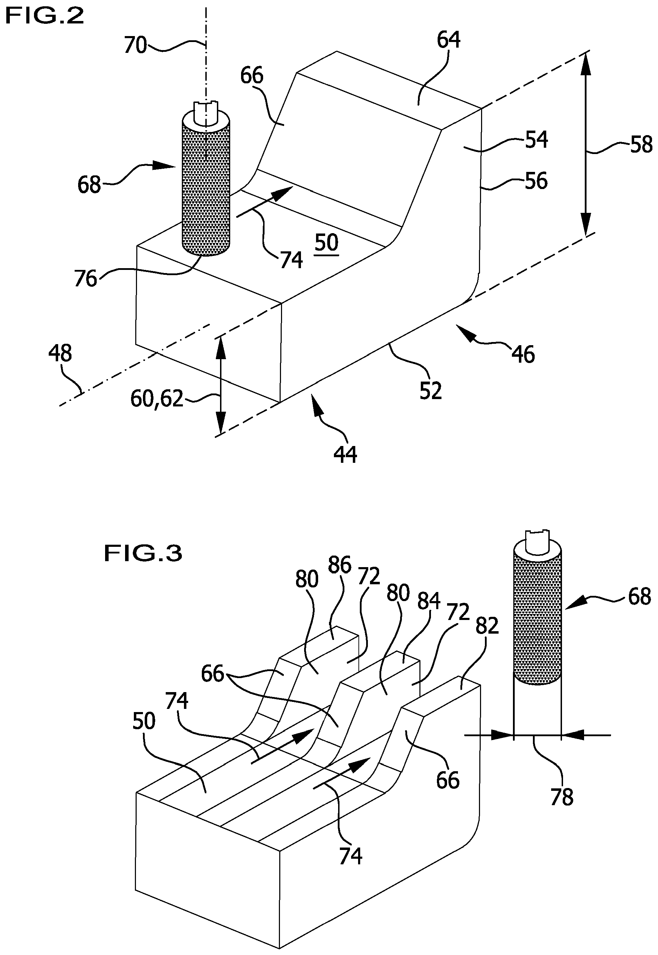

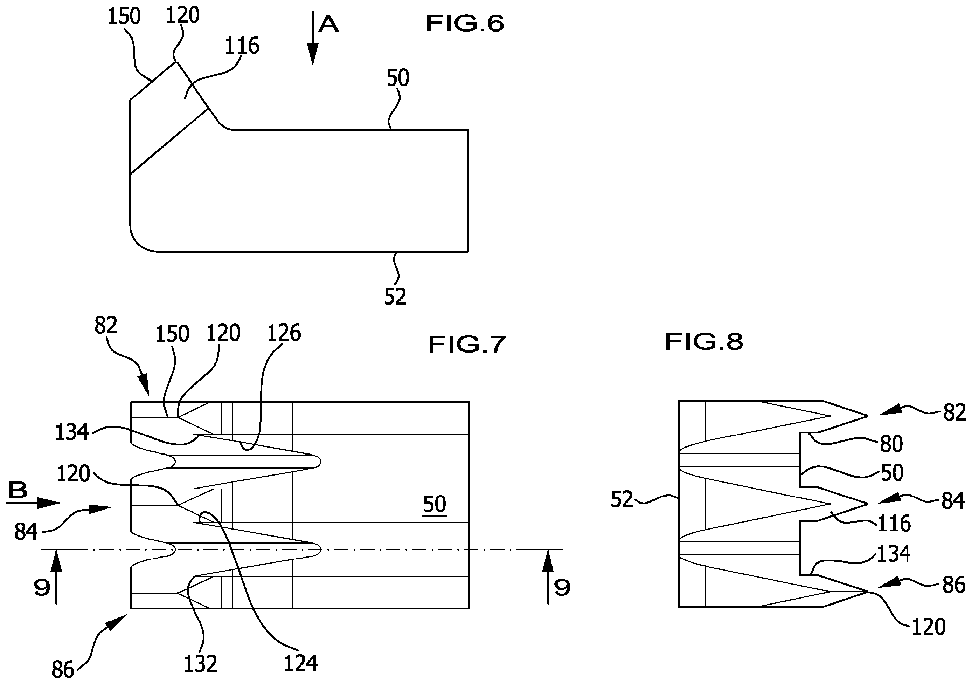

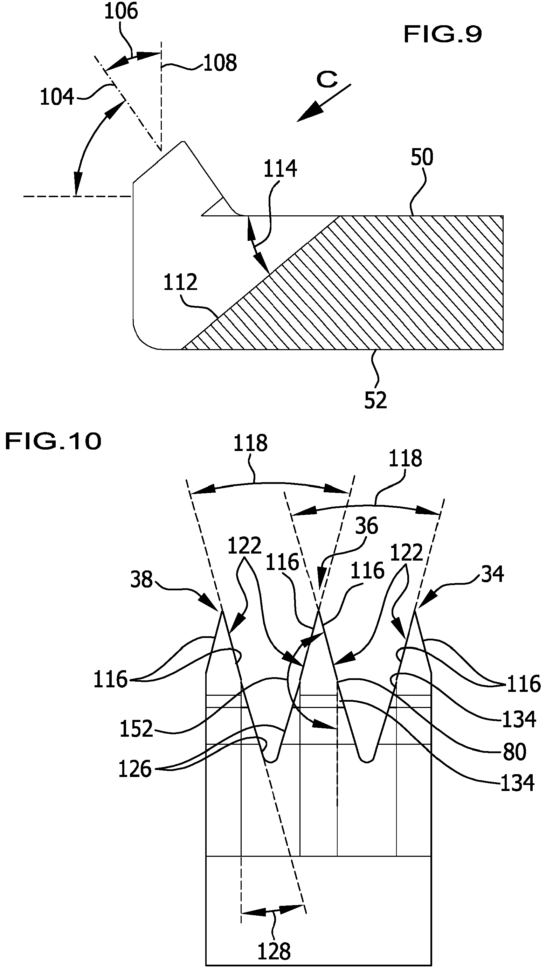

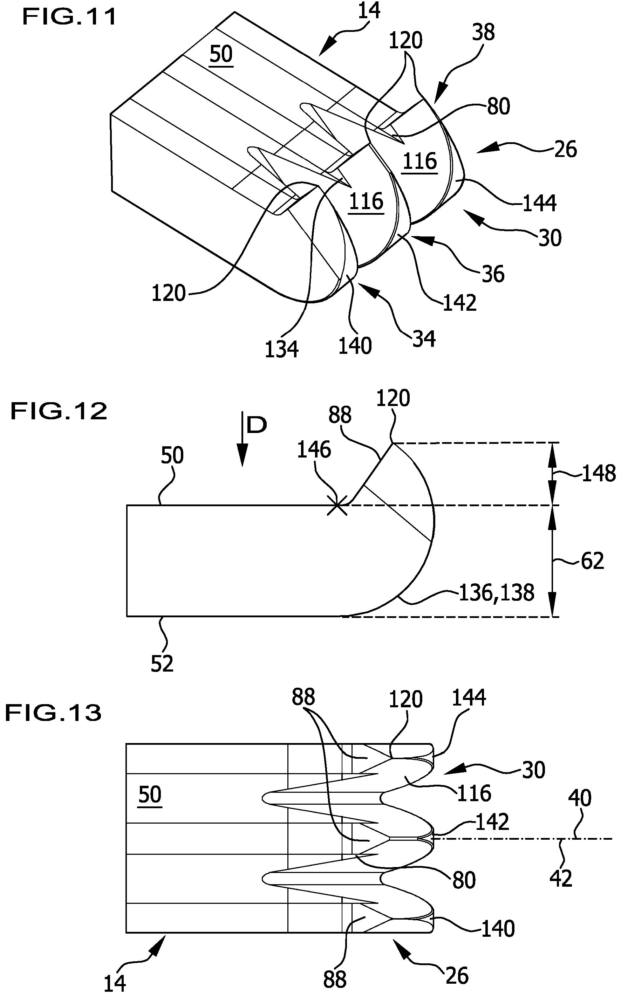

Nachfolgend wird beispielhaft die Ausbildung der zweiten Verzahnung 30 in Verbindung mit den Figuren 2 bis 13 beispielhaft erläutert.

Zur Ausbildung des zweiten Instrumentenarms 14 wird in einem ersten Schritt ein Instrumentenarmrohling 44 bereitgestellt, welcher einen distalen Endbereich 46 definiert, der eine Längsrichtung 48 definiert. Ein Querschnitt des Instrumentenarmrohlings 44 ist im Wesentlichen rechteckig und definiert eine Oberseite 50 sowie eine Unterseite 52. Die Oberseite 50 und die Unterseite 52 verlaufen im Wesentlichen parallel zueinander und weisen in voneinander gegengesetzte Richtungen.

Der Instrumentenarmrohling 44 ist aus einem metallischen Werkstoff ausgebildet und durch Kaltverformen in die beschriebene Form gebracht.

Von der Oberseite 50 abstehend ist ein Zahnvorsprung 54 ausgebildet, welcher quer zur Längsrichtung 48 von der Oberseite 50 absteht. Eine Endfläche 56 des Instrumentenarmrohlings 44 verläuft senkrecht zur Längsrichtung 48 und weist eine Höhe 58 auf, die etwa doppelt so groß ist wie ein Abstand 60 zwischen der Oberseite 50 und der Unterseite 52. Der Abstand 60 definiert mithin also eine Dicke 62 des Instrumentenarmrohlings 44.

Der Zahnvorsprung 54 weist eine parallel zur Oberseite verlaufende Zahnvorsprungoberseite 64 auf. Von dieser erstreckt sich in proximaler Richtung eine geneigte Zahnfläche 66 bis zur Oberseite 50.

In einem ersten Bearbeitungsschritt wird der Zahnvorsprung 54 mit einem Zylinderfräser 68 bearbeitet. Eine Längsachse 70 des Zylinderfräsers 68, um die der Zylinderfräser 68 zur Bearbeitung des Zahnvorsprungs 54 rotiert, verläuft senkrecht zur Oberseite 50.

Mit dem Zylinderfräser 68 werden am Zahnvorsprung 54 zwei identische Nuten 72 ausgebildet, und zwar indem der Zylinderfräser 68 in der beschriebenen Orientierung in Richtung des Pfeils 74 parallel zur Längsrichtung 48 bewegt wird. Eine in Richtung auf die Oberseite 50 weisende Stirnseite 76 des Zylinderfräsers 68 berührt die Oberseite 50, ohne diese jedoch zu bearbeiten.

Das Ergebnis dieses ersten Bearbeitungsschritts mit zwei Fräsvorgängen zur Ausbildung der Nuten 72 ist schematisch in Figur 3 dargestellt.

Der Zylinderfräser 68 weist einen Durchmesser 78 auf.

Die Nuten 72 werden von parallel zueinander verlaufenden und aufeinander zu weisenden Flächenbereichen 80 seitlich begrenzt sowie von der sich zwischen die drei verbleibenden Vorsprünge 82, 84 und 86 hinein verlängerte Oberseite 50.

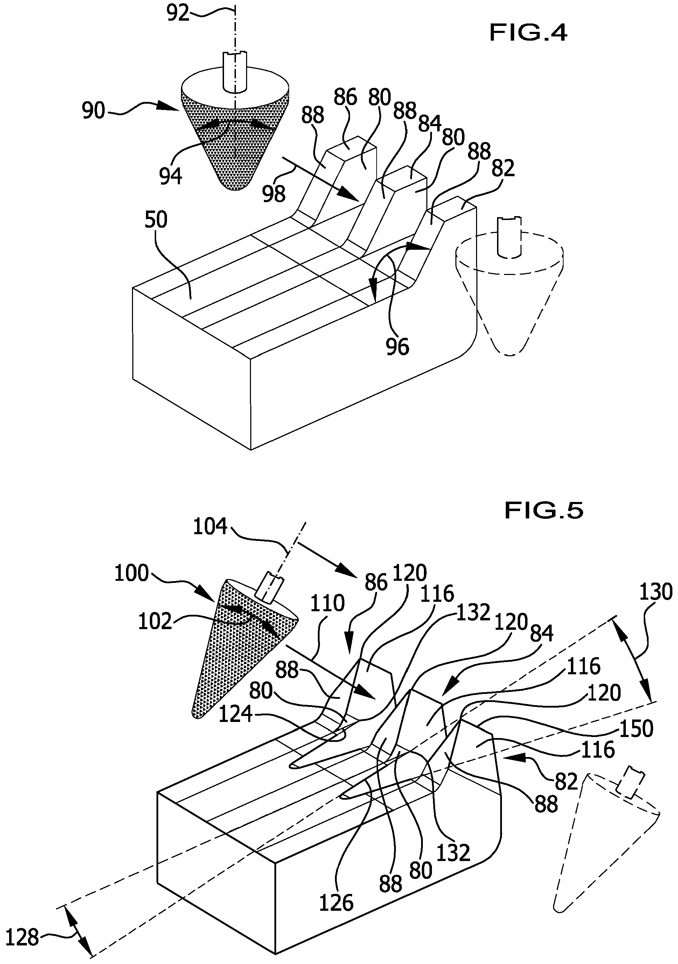

Ein optionaler Bearbeitungsschritt des Herstellungsverfahrens ist schematisch in Figur 4 dargestellt.

Um die Zahnfläche 66 in einer definierten Weise auszubilden, was durch Kaltverformen am Instrumentenarmrohling 44 nicht mit entsprechend hoher Reproduzierbarkeit sicher erreicht werden kann, werden im Sinne der Ansprüche dritte Flächenbereiche 88 mittels eines im Sinne der Ansprüche zweiten Kegelfräsers an den Vorsprüngen 82, 84 und 86 ausgebildet. Der zweite Kegelfräser 90 wird dazu mit seiner Längsachse 92 senkrecht zur Oberseite 50 ausgerichtet und an der Zahnfläche 66 entlang bewegt.

Der zweite Kegelfräser 90 weist einen Kegelwinkel 94 auf. Dieser beträgt bei dem in den Figuren dargestellten Ausführungsbeispiel etwa 70°. Ein Öffnungswinkel 96, der zwischen den dritten Flächenbereichen 88 und der Oberseite 50 definiert wird, beträgt etwa 125°. Dies entspricht einer Winkelsumme des halben Kegelwinkels 94 zuzüglich 90°.

Der zweite Kegelfräser 90 wird in einer Richtung symbolisiert durch den Pfeil 98 parallel zur Oberseite 50 bewegt, um die dritten Flächenbereiche 88 auszubilden.

Der in Verbindung mit Figur 4 dargestellte Bearbeitungsschritt ist wie bereits erwähnt optional. Falls die Zahnfläche 66 die vorgegebenen Spezifikationen erfüllt, kann auf die beschriebene Bearbeitung der Vorsprünge 82, 84 und 86 mit dem zweiten Kegelfräser 90 auch verzichtet werden.

Dieser optionale Bearbeitungsschritt mit dem zweiten Kegelfräser 90 kann alternativ auch vor dem Bearbeitungsschritt mit dem Zylinderfräser 68 durchgeführt werden.

Ein dritter Bearbeitungsschritt zur Ausbildung der zweiten Verzahnung 30 ist schematisch in Figur 5 dargestellt. In diesem Bearbeitungsschritt kommt im Sinne der Ansprüche ein erster Kegelfräser 100 zum Einsatz, welcher einen Kegelwinkel 102 definiert. Der Kegelwinkel 102 beträgt bei dem in den Figuren dargestellten Ausführungsbeispiel 30°.

Der erste Kegelfräser 100 definiert eine Längsachse 104, um die der erste Kegelfräser 100 zur Bearbeitung des distalen Endbereichs 46 rotiert wird.

Die Längsachse 104 ist beim Fräsen gegenüber der Oberseite 50 um einen Fräswinkel 106 geneigt. Der Fräswinkel 106 zwischen der Längsachse 104 und einer Flächennormalen 108 der Oberseite 50 beträgt bei dem in den Figuren dargestellten Ausführungsbeispiel 40°.

Der erste Kegelfräser 100 wird in einer Fräsrichtung symbolisiert durch den Pfeil 110 schräg nach vorne in distaler Richtung bewegt, so dass eine keilförmige Nut zwischen den Vorsprüngen 82 und 84 einerseits sowie 84 und 86 andererseits ausgebildet wird. Ein Nutgrund 112 verläuft parallel zur Fräsrichtung, also in Richtung des Pfeils 110, und ist relativ zur Oberseite 50 um einen Neigungswinkel 114 geneigt, welcher dem Fräswinkel 106 entspricht, mithin also etwa 40° bei dem in den Figuren dargestellten Ausführungsbeispiel.

Mit dem ersten Kegelfräser werden an den Vorsprüngen 82, 84 und 86 aufeinander zu weisende, im Sinne der Ansprüche zweite Flächenbereiche 116 ausgebildet. Aufeinander zu weisende zweite Flächenbereiche 116 definieren zwischen sich einen Keilwinkel 118, welcher dem Kegelwinkel 102 entspricht.

Die Zähne 34, 36 und 38 weisen nun nahezu ihre Endform auf. Zahnspitzen 120 bilden Schnittpunkte der beiden zweiten Flächenbereiche 116 sowie des dritten Flächenbereichs 88 der bearbeiteten Vorsprünge 82, 84 und 86.

Die Vorsprünge 82, 84 und 86 definieren aufeinander zu weisende Zahnflanken 122, die überwiegend durch einen zweiten Flächenbereich 116 sowie einen verbleibenden Rest eines ersten Flächenbereichs 80 gebildet werden.

Jeder erste Flächenbereich 80 definiert mit der Oberseite 50 eine erste Oberseitenschnittlinie 124.

Jeder zweite Flächenbereich 116 definiert mit der Oberseite 50 eine zweite Oberseitenschnittlinie 126.

Erste Oberseitenschnittlinien 124 und zweite Oberseitenschnittlinien 126 am selben Vorsprung 82, 84 beziehungsweise 86 schließen einen sich in proximaler Richtung öffnenden spitzen Winkel 128 ein.

Die zweiten Oberseitenschnittlinien 126 benachbarter Vorsprünge 82 beziehungsweise 84 und 86 definieren zwischen sich einen in distaler Richtung weisend geöffneten spitzen Winkel 130.

Ferner wird an jeder Zahnflanke 122 ein Schnittpunkt 132 definiert zwischen der ersten Oberseitenschnittlinie 124 und der zweiten Oberseitenschnittlinie 126, wobei sich in dem Schnittpunkt 132 der erste Flächenbereich 80, der zweite Flächenbereich 116 und die Oberseite 50 berühren.

Zudem weist jede Zahnflanke 122 einen Einschnitt 134 des zweiten Flächenbereichs 116 auf. Der Einschnitt 134 wird einerseits vom ersten Flächenbereich 80 und andererseits von der Oberseite 50 begrenzt.

In einem letzten Bearbeitungsschritt werden die Vorsprünge 82, 84 und 86 abgerundet. Dazu werden die verbleibenden Bereiche der Endfläche 56 so abgefräst oder abgeschliffen, dass eine Einhüllende 136 definiert wird, die einen Ausschnitt aus einer Zylinderfläche 138 bildet. Auf diese Weise werden in distaler Richtung weisende Endflächen 140, 142 und 144 der Zähne 34, 36 und 38 ausgebildet, die sich von der Unterseite 52 bis zur Zahnspitze 120 erstrecken.

Die Zylinderfläche 136 ist konzentrisch zu einer Zylinderlängsachse 146 ausgebildet, die sich parallel zur Oberseite 50 erstreckt und einen Teil derselben bildet.

Ein Abstand 148 der Zahnspitze von der Oberseite 50 ist kleiner als die Dicke 62.

Die zweite Verzahnung 30 umfasst wie beschrieben drei Zähne 34, 36 und 38. Die erste Verzahnung 28 umfasst zwei Zähne 32. Diese werden in analoger Weise wie der Zahn 36 ausgebildet. Die Form eines Zwischenraums zwischen den Zähnen 32 entspricht einer Form der Zwischenräume zwischen den Zähnen 34 und 36 beziehungsweise 36 und 38.

Die besondere Ausgestaltung der Verzahnungen 28 und 30 ermöglicht es, dass die Zähne 32 vollständig in die Zwischenräume zwischen den Zähnen 34 und 36 beziehungsweise 36 und 38 eingreifen können, und zwar so weit, dass die Oberseiten 50 der beiden Instrumentenarme 12 und 14 in der Schließstellung vollständig aneinander anliegen. Diese Eigenschaft wird insbesondere ermöglicht durch die Ausbildung der Einschnitte 134 an den Zahnflanken 122.

Die im Zusammenhang mit den Figuren 2, 3, 5 sowie im Ergebnis in den Figuren 14 und 15 dargestellten Bearbeitungsschritte können vollständig maschinell ausgeführt werden. Ebenso kann der in Verbindung mit Figur 4 beschriebene optionale Bearbeitungsschritt vollständig maschinell ausgeführt werden.

Zum Bearbeiten kann insbesondere eine CNC-Maschine eingesetzt werden, die die Fräswerkzeuge, nämlich den Zylinderfräser 68, die Kegelfräser 90 und 100 sowie ein in den Figuren nicht dargestelltes Fräswerkzeug zum Abrunden der Zähne 32, 34, 36 und 38 zur Ausbildung der Endflächen 140, 142 und 144 antreiben und bewegen kann.

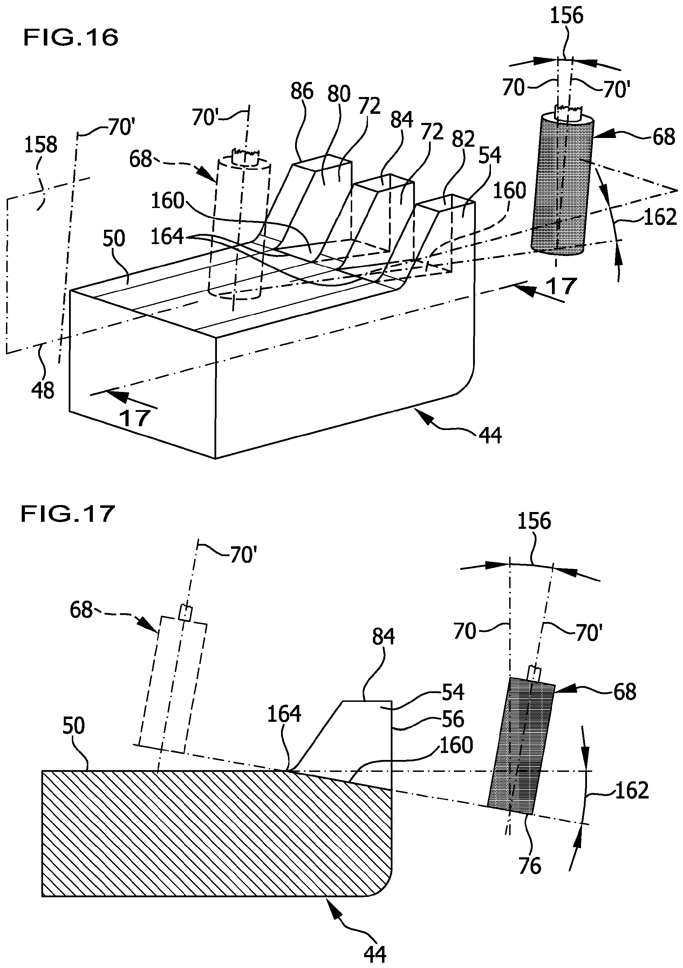

In den Figuren 16 bis 21 ist schematisch ein weiteres Ausführungsbeispiel eines medizinischen Instruments 10 dargestellt. Dieses weist eine große Ähnlichkeit zu dem in Verbindung mit den Figuren 1 bis 15 beschriebenen Ausführungsbeispiel auf, so dass zur Kennzeichnung identischer oder ähnlicher Teile und Komponenten identische Bezugszeichen verwendet wurden, teilweise mit einem Anstrich.

Exemplarisch ist in den Figuren 16 bis 19 dargestellt, wie aus einem Instrumentenarmrohling 44 einer der zwei Instrumentenarme 12 beziehungsweise 14 des Instruments 10 ausgebildet wird.

Der Instrumentenarmrohling 44, der schematisch in Figur 16 dargestellt ist, wurde bereits in einem ersten Bearbeitungsschritt mit dem zweiten Kegelfräser 90 bearbeitet, wie dies oben beispielhaft in Verbindung mit Figur 4 näher erläutert wurde.

In einem nächsten Bearbeitungsschritt wird der Zahnvorsprung 54 mit einem Zylinderfräser 68 bearbeitet. Anders als bei dem in den Figuren 2 und 3 oben beschriebenen Ausführungsbeispiel dargestellt und erläutert, ist die Längsachse 70' des Zylinderfräsers 80 um einen Fräserlängsachsenwinkel 156 bezogen auf die Fräserlängsachse 70, die eine Flächennormale zur Oberseite 50 definiert, geneigt. Die Längsachse 70' des Zylinderfräsers 68 definiert beim Fräsen mit der Längsrichtung 48 eine Fräsebene 158, welche senkrecht zur Oberseite 50 verläuft. Ferner ist die Längsachse 70' in distaler Richtung um den Fräserlängsachsenwinkel 156 geneigt.

In der beschriebenen Weise werden mit dem Zylinderfräser 68 am Zahnvorsprung 54 des Instrumentenarmrohlings 44 zwei identische Nuten 72 ausgebildet. Eine in Richtung auf die Oberseite 50 weisende Stirnseite 76 des Zylinderfräsers 68 bildet somit eine Schrägfläche 160 aus, die relativ zur Oberseite 50 um einen Schrägenwinkel 162 geneigt ist.

Die Schrägfläche 160 und die Oberseite 50 schneiden sich in einer Schrägflächenoberseitenschnittlinie 64, die quer, nämlich senkrecht, zur Längsrichtung 48 verläuft.

Die beschriebene Bearbeitung des Zahnvorsprungs 54 mit dem Zylinderfräser 68 ist schematisch in den Figuren 16 und 17 dargestellt.

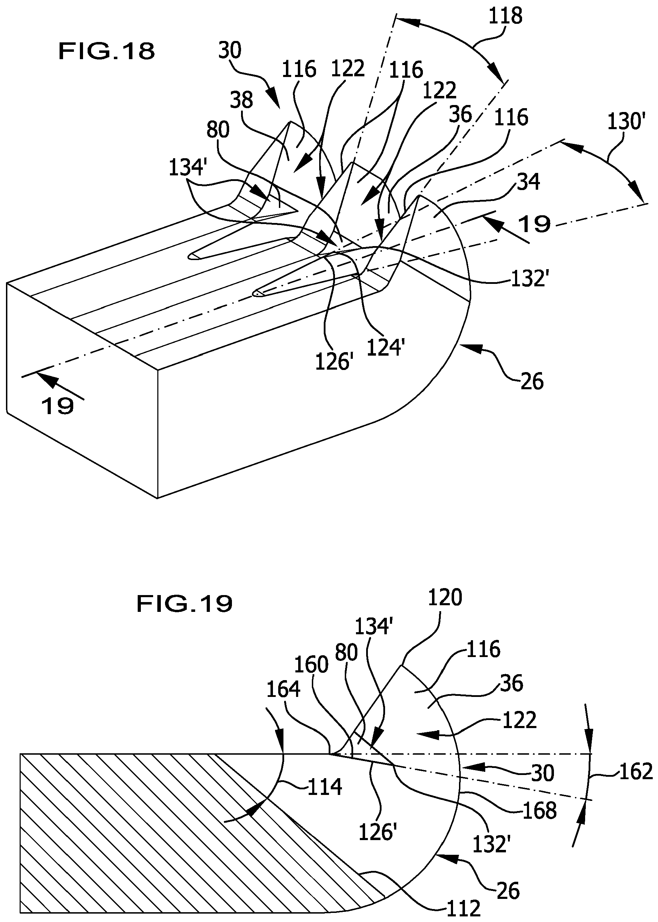

In einem weiteren Bearbeitungsschritt, welcher wahlweise vor oder nach der Bearbeitung mit dem Zylinderfräser 68 erfolgt, wird, und zwar in der oben in Verbindung mit Figur 5 beschriebenen Weise, zur Ausbildung der zweiten Verzahnung 30 der erste Kegelfräser 100 in einer Fräsrichtung symbolisiert durch den Pfeil 110 nach vorne in distaler Richtung bewegt, so dass eine keilförmige Nut zwischen den Vorsprüngen 82 und 84 einerseits sowie 84 und 86 andererseits ausgebildet wird. Ein Nutgrund 112 verläuft parallel zur Fräsrichtung, also in Richtung des Pfeils 110, und ist relativ zur Oberseite 50 um den Neigungswinkel 114 geneigt, welcher dem Fräswinkel 106 entspricht, mithin also etwa 40° bei dem in den Figuren 18 bis 21 dargestellten Ausführungsbeispiel.

Mit dem ersten Kegelfräser 100 werden auch bei diesem Ausführungsbeispiel an den Vorsprüngen 82, 84 und 86 aufeinander zu weisende, im Sinne der Ansprüche zweite Flächenbereiche 116 ausgebildet, die zwischen sich einen Keilwinkel 118 definieren, welcher dem Kegelwinkel 102 entspricht.

Der Schrägenwinkel 162 liegt in einem Bereich von etwa 3° bis etwa 30°. Bei dem in den Figuren 17 bis 21 dargestellten Ausführungsbeispiel beträgt der Schrägenwinkel etwa 10°.

Auch bei diesem Ausführungsbeispiel definieren die Vorsprünge 82, 84 und 86 aufeinander zu weisende Zahnflanken 122, die überwiegend durch einen zweiten Flächenbereich 116 sowie einen verbleibenden Rest eines ersten Flächenbereichs 80 gebildet werden.

Der erste Flächenbereich 80 und die Schrägfläche 160 definieren eine erste Schrägflächenschnittlinie 124'. Ferner definiert der zweite Flächenbereich 116 mit der Schrägfläche 160 eine zweite Schrägflächenschnittlinie 126'.

Die erste Schrägflächenschnittlinie 124' und die zweite Schrägflächenschnittlinie 126' derselben Zahnflanke 120 schließen einen sich in proximaler Richtung öffnenden spitzen Winkel 128' ein.

Die zweiten Schrägflächenschnittlinien 126' benachbarter Zähne der Verzahnung definieren einen in distaler Richtung weisend geöffneten spitzen Winkel 130'.

Ferner wird an jeder Zahnflanke 122 ein Schnittpunkt 132' zwischen der ersten Oberseitenschnittlinie 124' und der zweiten Oberseitenschnittlinie 126' definiert, wobei sich im Schnittpunkt 132' der erste Flächenbereich 80, der zweite Flächenbereich 116 und die Schrägfläche 160 berühren.

Ferner weist jede Zahnflanke 122 einen Einschnitt 134' des zweiten Flächenbereichs 116 auf. Der Einschnitt 134' wird einerseits vom ersten Flächenbereich 80 und andererseits von der Schrägfläche 160 begrenzt.

Die Bearbeitungsschritte mit dem Zylinderfräser 68 und dem ersten Kegelfräser 100, können alternativ auch in vertauschter Reihenfolge durchgeführt werden. Dies gilt entsprechend auch für das in den Figuren 2 bis 15 schematisch dargestellte Ausführungsbeispiel. Im Ergebnis ändert dies an der Form der darin ausgebildeten Verzahnungen 28 beziehungsweise 30 nichts.

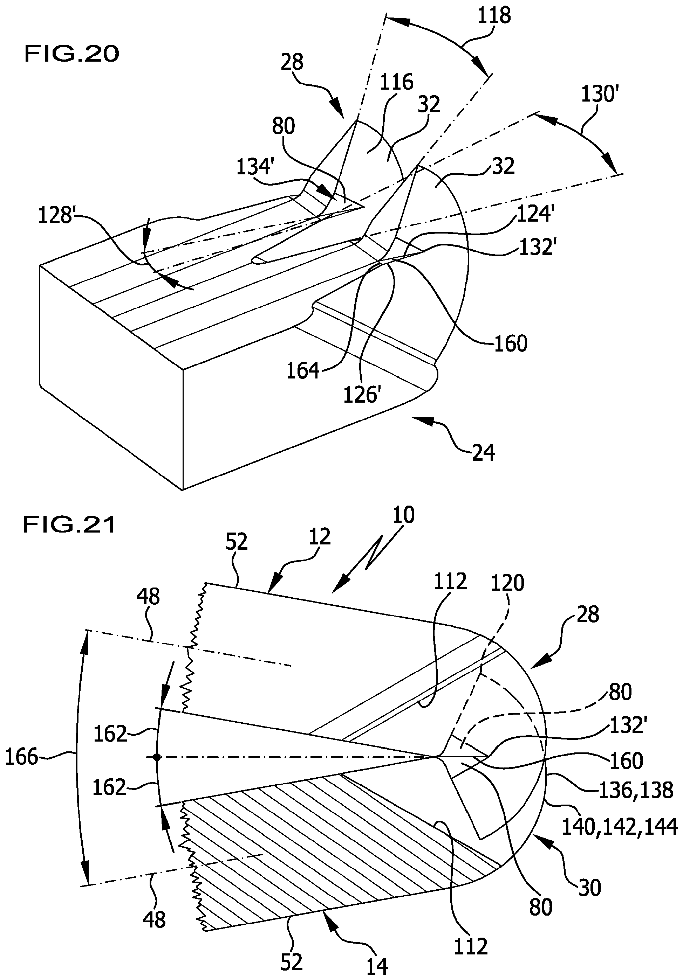

In Figur 20 ist schematisch ein distales Ende 24 des ersten Instrumentenarms 12 dargestellt. Ein distales Ende 26 des zweiten Instrumentenarms 14 zeigt schematisch Figur 18.

Die Ausbildung der Schrägflächen 160 ermöglicht es, wie schematisch in Figur 21 dargestellt, die Instrumentenarme 12 und 14 mit ihren Längsrichtungen 48 gegeneinander zu neigen, und zwar um einen Neigungswinkel 166. Die Schrägflächen 160 können dann noch flächig in Anlage gebracht werden, wenn der Neigungswinkel 160 dem doppelten des Schrägenwinkels 162 entspricht. Die distalen Enden 24 und 26 der Instrumentenarme 12 und 14 des Instruments 10 sind dann so ausgebildet, dass sie gut und ohne zu verhaken oder zu verklemmen ineinandergreifen, wenn die Längsrichtungen 48 der beiden Instrumentenarme 12 und 14 einen Neigungswinkel 166 einschließen, der nicht größer als das Doppelte des Schrägenwinkels 162 ist.

Die Endflächen 140, 142 und 144 der Zähne 34, 36 und 38 werden auch bei dem in den Figuren 16 bis 21 dargestellten Ausführungsbeispiel verrundet beziehungsweise abgerundet. Dazu werden die verbleibenden Bereiche der ursprünglichen Endfläche 56 des Zahnvorsprungs 54 so abgefräst oder abgeschliffen, dass eine Einhüllende 136 definiert wird, die einen Ausschnitt aus einer Kugeloberfläche 168 bildet. So werden in distaler Richtung weisende Endflächen 140, 142 und 144 der Zähne 34, 36 und 38 ausgebildet, die sich von der Unterseite 52 bis zur Zahnspitze 120 erstrecken.

Auch die im Zusammenhang mit den Figuren 16 bis 21 beschriebenen Bearbeitungsschritte können vollständig maschinell ausgeführt werden, beispielsweise mit einer CNC-Maschine, welche die Fräswerkzeuge, nämlich den Zylinderfräser 68, die Kegelfräser 90 und 100 sowie ein in den Figuren nicht dargestelltes Fräswerkzeug zum Abrunden der Zähne 32, 34, 36 und 38 zur Ausbildung der Endflächen 140, 142 und 144 antreiben und bewegen kann.

In der beschriebenen Weise lässt sich ein medizinisches Instrument, insbesondere in Form einer Pinzette, aus zwei Instrumentenarmrohlingen durch maschinelle Bearbeitung in reproduzierbarer Weise mit gleichbleibender Qualität ausbilden.

### Bezugszeichenliste

- 10: Instrument
- 12: erster Instrumentenarm
- 14: zweiter Instrumentenarm
- 16: Endbereich
- 18: Verbindungselement
- 20: Endabschnitt
- 22: Endabschnitt
- 24: distales Ende
- 26: distales Ende
- 28: erste Verzahnung
- 30: zweite Verzahnung
- 32: Zahn
- 34: Zahn
- 36: Zahn
- 38: Zahn
- 40: Verzahnungsspiegelebene
- 42: Zahnspiegelebene
- 44: Instrumentenarmrohling
- 46: Endbereich
- 48: Längsrichtung
- 50: Oberseite
- 52: Unterseite
- 54: Zahnvorsprung
- 56: Endfläche
- 58: Höhe
- 60: Abstand
- 62: Dicke
- 64: Zahnvorsprungoberseite
- 66: Zahnfläche
- 68: Zylinderfräser
- 70, 70': Längsachse
- 72: Nut
- 74: Pfeil
- 76: Stirnseite
- 78: Durchmesser
- 80: erster Flächenbereich
- 82: Vorsprung
- 84: Vorsprung
- 86: Vorsprung
- 88: dritter Flächenbereich
- 90: zweiter Kegelfräser
- 92: Längsachse
- 94: Kegelwinkel
- 96: Öffnungswinkel
- 98: Pfeil
- 100: erster Kegelfräser
- 102: Kegelwinkel
- 104: Längsachse
- 106: Fräswinkel
- 108: Flächennormale
- 110: Pfeil
- 112: Nutgrund
- 114: Neigungswinkel
- 116: zweiter Flächenbereich
- 118: Keilwinkel
- 120: Zahnspitze
- 122: Zahnflanke
- 124, 124': erste Oberseitenschnittlinie
- 126, 126': zweite Oberseitenschnittlinie
- 128, 128': Winkel
- 130, 130': Winkel
- 132, 132': Schnittpunkt
- 134, 134': Einschnitt
- 136: Einhüllende
- 138: Zylinderfläche
- 140: Endfläche
- 142: Endfläche
- 144: Endfläche
- 146: Zylinderlängsachse
- 148: Abstand
- 150: Schnittlinie
- 152: Flächenbereichswinkel
- 154: Pinzette
- 156: Fräserlängsachsenwinkel
- 158: Fräserebene
- 160: Schrägfläche
- 162: Schrägenwinkel
- 164: Schrägflächenoberseitenschnittlinie
- 166: Neigungswinkel
- 168: Kugeloberfläche

## Patentansprüche

1. Medizinisches Instrument (10) mit mindestens einem Instrumentenarm (12; 14), wobei an einem distalen Ende (24; 26) des Instrumentenarms (12; 14) eine Verzahnung (28; 30) mit mindestens zwei Zähnen (32; 34, 36, 38) ausgebildet ist, die quer vom distalen Ende (24; 26) abstehen, wobei jeder der mindestens zwei Zähne (32; 34, 36, 38) zwei voneinander weg weisende Zahnflanken (122) aufweist, wobei jede Zahnflanke (122) mindestens einen ebenen ersten Flächenbereich (80) und mindestens einen ebenen zweiten Flächenbereich (116) aufweist, wobei der mindestens eine ebene erste Flächenbereich (80) und der mindestens eine ebene zweite Flächenbereich (116) um einen Flächenbereichswinkel (152) gegeneinander geneigt sind und wobei die ersten und zweiten Flächenbereiche (80, 116) durch Fräsen, insbesondere mit einer CNC-Maschine, ausgebildet sind.

2. Medizinisches Instrument nach Anspruch 1, **dadurch gekennzeichnet, dass** ein distaler Endbereich (16) des mindestens einen Instrumentenarms (12, 14) eine Längsrichtung (48) definiert und dass die Verzahnung (28; 30) bezogen auf die Längsrichtung (48) quer abstehend ausgebildet ist.

3. Medizinisches Instrument nach einem der voranstehenden Ansprüche,
**dadurch gekennzeichnet, dass** der Instrumentenarm (12; 14), insbesondere dessen distaler Endbereich (16), eine ebene Oberseite (50) aufweist und dass die Verzahnung (28; 30) mindestens teilweise über die Oberseite (50) vorsteht,
wobei insbesondere jede Zahnflanke (122) einen Einschnitt (134) des zweiten Flächenbereichs (116) aufweist und wobei der Einschnitt (134) einerseits vom ersten Flächenbereich (80) und andererseits von der Oberseite (50) begrenzt ist.

4. Medizinisches Instrument nach Anspruch 3, **dadurch gekennzeichnet, dass** jeder erste Flächenbereich (80) mit der Oberseite (50) eine erste Oberseitenschnittlinie (124) definiert und dass jeder zweite Flächenbereich (116) mit der Oberseite (50) eine zweite Oberseitenschnittlinie (126) definiert,
wobei insbesondere
a) die erste Oberseitenschnittlinie (124) und die zweite Oberseitenschnittlinie (126) derselben Zahnflanke (122) einen sich in proximaler Richtung öffnenden spitzen Winkel (128) einschließen
und/oder
b) die zweiten Oberseitenschnittlinien (126) benachbarter Zähne (32; 34, 36; 36, 38) der Verzahnung (28; 30) einen sich in distaler Richtung weisend geöffneten spitzen Winkel (130) definieren
und/oder
c) sich in einem Schnittpunkt (132) der ersten Oberseitenschnittlinie (124) und der zweiten Oberseitenschnittlinie (126) der erste Flächenbereich (80) und der zweite Flächenbereich (116) einer Zahnflanke (122) und die Oberseite (50) berühren
und/oder
d) die erste Oberseitenschnittlinie (124) parallel oder im Wesentlichen parallel zur Längsrichtung (48) verläuft.

5. Medizinisches Instrument nach Anspruch 4, **dadurch gekennzeichnet, dass** jeder erste Flächenbereich (80) mit einer Schrägfläche (160), welche relativ zur Oberseite (50) um einen Schrägenwinkel (162) geneigt ist, eine erste Schrägflächenschnittlinie (124') definiert und dass jeder zweite Flächenbereich (116) mit der Schrägfläche (160) eine zweite Schrägflächenschnittlinie (126') definiert,
wobei insbesondere
a) der Schrägenwinkel (162) einen Wert in einem Bereich von etwa 3° bis etwa 30° aufweist, insbesondere in einem Bereich von etwa 8° bis etwa 13°, weiter insbesondere einen Wert von etwa 10° aufweist,
und/oder
b) sich die Schrägfläche (160) und die Oberseite (50) in einer Schrägflächenoberseitenschnittlinie (164) schneiden, die quer, insbesondere senkrecht, zur Längsrichtung verläuft
und/oder
c) die erste Schrägflächenschnittlinie (124') und die zweite Schrägflächenschnittlinie (126') derselben Zahnflanke (122) einen sich in proximaler Richtung öffnenden spitzen Winkel (128') einschließen
und/oder
d) die zweiten Schrägflächenschnittlinien (126') benachbarter Zähne (32; 34, 36; 36, 38) der Verzahnung (28; 30) einen in distaler Richtung weisend geöffneten spitzen Winkel (130') definieren
und/oder
e) sich in einem Schnittpunkt (132') der ersten Schrägflächenschnittlinie (124') und der zweiten Schrägflächenschnittlinie (126') der erste Flächenbereich (80) und der zweite Flächenbereich (116) einer Zahnflanke (122) und die Schrägfläche (160) berühren
und/oder
f) die erste Schrägflächenschnittlinie (124') und die Längsrichtung (48) um den Schrägenwinkel (162) gegeneinander geneigt sind.

6. Medizinisches Instrument nach einem der voranstehenden Ansprüche,
**dadurch gekennzeichnet, dass** das Instrument (10)
a) zwei Instrumentenarme (12, 14) umfasst, dass an einem ersten Instrumentenarm (12) der zwei Instrumentenarme (12, 14) eine erste Verzahnung (28) ausgebildet ist, die in Richtung auf einen zweiten Instrumentenarm (14) der zwei Instrumentenarme (12, 14) hin weist, dass am zweiten Instrumentenarm (14) eine zweite Verzahnung (30) ausgebildet ist, die in Richtung auf den ersten Instrumentenarm (12) hin weist und dass in einer Schließstellung des Instruments (10), in welcher distale Enden (24, 26) der zwei Instrumentenarme (12, 14) einander maximal angenähert sind, die erste Verzahnung (28) und die zweite Verzahnung (30) ineinandergreifen, wobei die zweite Verzahnung (30) einen Zahn (34, 36, 38) mehr umfasst als die erste Verzahnung (28),
und/oder
b) in Form einer Pinzette (154) ausgebildet ist.

7. Verfahren zum Herstellen eines medizinisches Instrument (10), welches mindestens einen Instrumentenarm (12; 14) umfasst, wobei an einem distalen Ende (24; 26) des Instrumentenarms (12; 14) eine Verzahnung (28; 30) mit mindestens zwei Zähnen (32; 34, 36, 38) ausgebildet wird, die quer vom distalen Ende (24; 26) abstehen, wobei jeder der mindestens zwei Zähne (32; 34, 36, 38) zwei voneinander weg weisende Zahnflanken (122) aufweist, wobei jede Zahnflanke (122) mit mindestens einem ebenen ersten Flächenbereich (80) und mindestens einem ebenen zweiten Flächenbereich (116) ausgebildet wird, wobei der mindestens eine ebene erste Flächenbereich (80) und der mindestens eine ebene zweite Flächenbereich (116) um einen Flächenbereichswinkel (152) gegeneinander geneigt sind und wobei die ersten und zweiten Flächenbereiche (80, 116) durch Fräsen, insbesondere mit einer CNC-Maschine, ausgebildet werden.

8. Verfahren nach Anspruch 7, **dadurch gekennzeichnet, dass** ein distaler Endbereich (16) des mindestens einen Instrumentenarms (12; 14) eine Längsrichtung (48) definiert und dass die Verzahnung (28; 30) bezogen auf die Längsrichtung (48) quer abstehend ausgebildet wird.

9. Verfahren nach Anspruch 7 oder 8, **dadurch gekennzeichnet, dass** der Instrumentenarm (12; 14), insbesondere dessen distaler Endbereich (16), eine ebene Oberseite (50) aufweist und dass die Verzahnung (28; 30) mindestens teilweise über die Oberseite (50) vorstehend ausgebildet wird,
wobei insbesondere die Zähne (32; 34, 36, 38) derart ausgebildet werden, dass jede Zahnflanke (122) einen Einschnitt (134) des zweiten Flächenbereichs (116) aufweist und dass der Einschnitt (134) einerseits vom ersten Flächenbereich (80) und andererseits von der Oberseite (50) begrenzt ist.

10. Verfahren nach Anspruch 9, **dadurch gekennzeichnet, dass** jeder erste Flächenbereich (80) derart ausgebildet wird, dass er mit der Oberseite (50) eine erste Oberseitenschnittlinie (124) definiert, und dass jeder zweite Flächenbereich (116) derart ausgebildet wird, dass er mit der Oberseite (50) eine zweite Oberseitenschnittlinie (126) definiert, wobei insbesondere
a) jede Zahnflanke (122) derart ausgebildet wird, dass die erste Oberseitenschnittlinie (124) und die zweite Oberseitenschnittlinie (126) einen sich in proximaler Richtung öffnenden spitzen Winkel (128) einschließen
und/oder
b) die Verzahnung (28; 30) derart ausgebildet wird, dass die zweiten Oberseitenschnittlinien (126) benachbarter Zähne (32; 34, 36; 36, 38) einen in distaler Richtung weisend geöffneten spitzen Winkel (130) definieren
und/oder
c) die Verzahnung (28; 30) derart ausgebildet wird, dass sich in einem Schnittpunkt (132) der ersten Oberseitenschnittlinie (124) und der zweiten Oberseitenschnittlinie (126) der erste Flächenbereich (80) und der zweite Flächenbereich (116) einer Zahnflanke (122) und die Oberseite (50) berühren
und/oder
d) Zähne (32; 34, 36, 38) derart ausgebildet werden, dass die erste Oberseitenschnittlinie (124) parallel oder im Wesentlichen parallel zur Längsrichtung (80) verläuft.

11. Verfahren nach Anspruch 9, **dadurch gekennzeichnet, dass** jeder erste Flächenbereich (80) derart ausgebildet wird, dass er mit einer Schrägfläche (160), welche relativ zur Oberseite (50) um einen Schrägenwinkel (162) geneigt ist, eine erste Schrägflächenschnittlinie (124') definiert und dass jeder zweite Flächenbereich (116) derart ausgebildet wird, dass er mit der Schrägfläche (160) eine zweite Schrägflächenschnittlinie (126') definiert.

12. Verfahren nach Anspruch 11, **dadurch gekennzeichnet, dass**
a) der Schrägenwinkel (162) mit einem Wert in einem Bereich von etwa 3° bis etwa 30° ausgebildet wird, insbesondere mit einem Wert in einem Bereich von etwa 8° bis etwa 13°, weiter insbesondere mit einem Wert von etwa 10°,
und/oder
b) jeder erste Flächenbereich (80) derart ausgebildet wird, dass sich die Schrägfläche (160) und die Oberseite (50) in einer Schrägflächenoberseitenschnittlinie (164) schneiden, die quer, insbesondere senkrecht, zur Längsrichtung (48) verläuft
und/oder
c) jede Zahnflanke (122) derart ausgebildet wird, dass die erste Schrägflächenschnittlinie (124') und die zweite Schrägflächenschnittlinie (126') derselben Zahnflanke (122) einen sich in proximaler Richtung öffnenden spitzen Winkel (128') einschließen
und/oder
d) die Verzahnung (28; 30) derart ausgebildet wird, dass die zweiten Schrägflächenschnittlinien (126') benachbarter Zähne (32; 34, 36; 36, 38) der Verzahnung (28; 30) einen in distaler Richtung weisend geöffneten spitzen Winkel (130') definieren
und/oder
e) die Verzahnung (28; 30) derart ausgebildet wird, dass sich in einem Schnittpunkt (132') der ersten Schrägflächenschnittlinie (124') und der zweiten Schrägflächenschnittlinie (126') der erste Flächenbereich (80) und der zweite Flächenbereich (116) einer Zahnflanke (122) und die Schrägfläche (160) berühren
und/oder
f) Zähne (32; 34, 36, 38) derart ausgebildet werden, dass die erste Schrägflächenschnittlinie (124') und die Längsrichtung (48) um den Schrägenwinkel (162) gegeneinander geneigt sind.

13. Verfahren nach einem der Ansprüche 7 bis 12, **dadurch gekennzeichnet, dass** der mindestens eine erste Flächenbereich (80) mit einem ersten Fräswerkzeug (68), insbesondere einem Zylinderfräser (68), ausgebildet wird,
wobei insbesondere eine Längsachse (70; 70') des Zylinderfräsers (68) beim Fräsen
a) sowohl senkrecht zur Oberseite (50) als auch senkrecht zur Längsrichtung (48) orientiert ist
oder
b) mit der Längsrichtung (48) eine Fräsebene (158) definiert, welche senkrecht zur Oberseite (50) verläuft, und in distaler Richtung um einen Fräserlängsachsenwinkel (156) geneigt ist,
wobei insbesondere der Fräserlängsachsenwinkel (156) dem Schrägenwinkel (162) entspricht.

14. Verfahren nach einem der Ansprüche 7 bis 13, **dadurch gekennzeichnet, dass**
a) der mindestens eine ebene zweite Flächenbereich (116) mit einem zweiten Fräswerkzeug (100), insbesondere einem ersten Kegelfräser (100), ausgebildet wird, wobei insbesondere eine Längsachse (104) des ersten Kegelfräsers (100) beim Fräsen relativ zur Oberseite (50) um einen in distaler Richtung geöffneten Neigungswinkel (114) geneigt ist und mit der Längsrichtung (48) eine zur Oberseite (50) senkrecht verlaufende Ebene definiert,
und/oder
b) jeder Zahn (32; 34, 36, 38) mit mindestens einem ebenen dritten Flächenbereich (88) ausgebildet wird,
wobei insbesondere der mindestens eine dritte Flächenbereich (88) durch Kaltverformen oder durch Fräsen, insbesondere mit einer CNC-Maschine, ausgebildet wird,
wobei weiter insbesondere der mindestens eine dritte Flächenbereich (88) mit einem dritten Fräswerkzeug (90), insbesondere einem zweiten Kegelfräser (90), ausgebildet wird, wobei insbesondere eine Längsachse (92) des zweiten Kegelfräsers (90) beim Fräsen sowohl senkrecht zur Oberseite (50) als auch senkrecht zur Längsrichtung (48) orientiert ist.

15. Verfahren nach einem der Ansprüche 10 bis 14,
**dadurch gekennzeichnet, dass**
a) die Verzahnung (28; 30) derart ausgebildet wird, dass jeder zweite Flächenbereich (116) eine zweite Flächenbereichebene definiert, dass die zweiten Flächenbereichebenen benachbarter Zähne (32; 34, 36; 36, 38) eine gemeinsame Flächenbereichschnittlinie definieren und dass die Flächenbereichschnittlinie mit der Längsrichtung (48) einen in distaler Richtung geöffneten Neigungswinkel (114) einschließt,
wobei insbesondere der Neigungswinkel (114) durch eine Orientierung der Längsachse (92) des zweiten Kegelfräsers (90) beim Fräsen relativ zur Oberseite (50) vorgegeben wird und wobei die Längsachse (92) senkrecht zur Flächenbereichschnittlinie ausgerichtet wird und mit der Längsrichtung (48) eine zur Oberseite (50) senkrecht verlaufende Ebene definiert, wobei insbesondere der Neigungswinkel (114) einen Wert in einem Bereich von etwa 30° bis etwa 50° aufweist, insbesondere etwa 40°,
und/oder
b) das Instrument (10) mit zwei Instrumentenarmen (12, 14) ausgebildet wird derart, dass an einem ersten Instrumentenarm (12) der zwei Instrumentenarme (12, 14) eine erste Verzahnung (28) ausgebildet wird, die in Richtung auf einen zweiten Instrumentenarm (14) der zwei Instrumentenarme (12, 14) hin weist, dass am zweiten Instrumentenarm (14) eine zweite Verzahnung (30) ausgebildet wird, die in Richtung auf den ersten Instrumentenarm (12) hin weist und dass in einer Schließstellung des Instruments (10), in welcher distale Enden (24, 26) der zwei Instrumentenarme (12, 14) einander maximal angenähert sind, die erste Verzahnung (28) und die zweite Verzahnung (30) ineinandergreifen,
wobei insbesondere die zweite Verzahnung (30) mit einem Zahn (34, 36, 38) mehr als die erste Verzahnung (28) ausgebildet wird.

## Claims

1. Medical instrument (10) having at least one instrument arm (12; 14), wherein formed on a distal end (24; 26) of the instrument arm (12; 14) is a toothing (28; 30) with at least two teeth (32; 34, 36, 38), which project transversely from the distal end (24; 26), wherein each of the at least two teeth (32; 34, 36, 38) has two tooth flanks (122) facing away from one another, wherein each tooth flank (122) has at least one planar first surface region (80) and at least one planar second surface region (116), wherein the at least one planar first surface region (80) and the at least one planar second surface region (116) are inclined toward one another by a surface region angle (152), and wherein the first and second surface regions (80, 116) are formed by milling, in particular with a CNC machine.

2. Medical instrument according to claim 1, **characterized in that** a distal end region (16) of the at least one instrument arm (12, 14) defines a longitudinal direction (48), and **in that** the toothing (28; 30) is configured projecting transversely relative to the longitudinal direction (48).

3. Medical instrument according to any one of the preceding claims, **characterized in that** the instrument arm (12; 14), in particular its distal end region (16), has a planar top side (50) and **in that** the toothing (28; 30) projects at least partially beyond the top side (50),
wherein in particular each tooth flank (122) has a notch (134) in the second surface region (116) and wherein the notch (134) is delimited by the first surface region (80) on the one hand and by the top side (50) on the other hand.

4. Medical instrument according to claim 3, **characterized in that** each first surface region (80) together with the top side (50) defines a first top side intersection line (124) and **in that** each second surface region (116) together with the top side (50) defines a second top side intersection line (126),
wherein in particular
a) the first top side intersection line (124) and the second top side intersection line (126) of the same tooth flank (122) enclose an acute angle (128) opening in the proximal direction
and/or
b) the second top side intersection lines (126) of adjacent teeth (32; 34, 36; 36, 38) of the toothing (28; 30) define an acute angle (130) opened pointing in the distal direction
and/or
c) the first surface region (80) and the second surface region (116) of a tooth flank (122) and the top side (50) come into contact at a point of intersection (132) of the first top side intersection line (124) and the second top side intersection line (126)
and/or
d) the first top side intersection line (124) extends in parallel or substantially in parallel to the longitudinal direction (48).

5. Medical instrument according to claim 4, **characterized in that** each first surface region (80) together with a sloped face (160), which is inclined relative to the top side (50) by a slope angle (162), defines a first sloped face intersection line (124') and **in that** each second surface region (116) together with the sloped face (160) defines a second sloped face intersection line (126'),
wherein in particular
a) the slope angle (162) has a value in a range of about 3° to about 30°, in particular in a range of about 8° to about 13°, further in particular has a value of about 10°,
and/or
b) the sloped face (160) and the top side (50) intersect in a sloped face top side intersection line (164), which extends transversely, in particular perpendicularly, to the longitudinal direction
and/or
c) the first sloped face intersection line (124') and the second sloped face intersection line (126') of the same tooth flank (122) enclose an acute angle (128') opening in the proximal direction
and/or
d) the second sloped face intersection lines (126') of adjacent teeth (32; 34, 36; 36, 38) of the toothing (28; 30) define an acute angle (130') opened pointing in the distal direction
and/or
e) the first surface region (80) and the second surface region (116) of a tooth flank (122) and the sloped face (80) come into contact at a point of intersection (132') of the first sloped face intersection line (124') and the second sloped face intersection line (126')
and/or
f) the first sloped face intersection line (124') and the longitudinal direction (48) are inclined toward one another by the slope angle (162).

6. Medical instrument according to any one of the preceding claims, **characterized in that** the instrument (10)
a) comprises two instrument arms (12, 14), **in that** a first toothing (28) is formed on a first instrument arm (12) of the two instrument arms (12, 14), the first toothing pointing in the direction toward a second instrument arm (14) of the two instrument arms (12, 14), **in that** a second toothing (30) is formed on the second instrument arm (14), the second toothing pointing in the direction toward the first instrument arm (12), and **in that** in a closing position of the instrument (10) in which distal ends (24, 26) of the two instrument arms (12, 14) are maximally proximate to one another, the first toothing (28) and the second toothing (30) interengage, wherein the second toothing (30) comprises one more tooth (34, 36, 38) than the first toothing (28),
and/or
b) is configured in the form of forceps (154).

7. Method for producing a medical instrument (10), which comprises at least one instrument arm (12; 14), wherein formed on a distal end (24; 26) of the instrument arm (12; 14) is a toothing (28; 30) with at least two teeth (32; 34, 36, 38), which project transversely from the distal end (24; 26), wherein each of the at least two teeth (32; 34, 36, 38) has two tooth flanks (122) facing away from one another, wherein each tooth flank (122) is configured having at least one planar first surface region (80) and at least one planar second surface region (116), wherein the at least one planar first surface region (80) and the at least one planar second surface region (116) are inclined toward one another by a surface region angle (152), and wherein the first and second surface regions (80, 116) are formed by milling, in particular with a CNC machine.

8. Method according to claim 7, **characterized in that** a distal end region (16) of the at least one instrument arm (12; 14) defines a longitudinal direction (48) and **in that** the toothing (28; 30) is configured projecting transversely relative to the longitudinal direction (48).

9. Method according to claim 7 or 8, **characterized in that** the instrument arm (12; 14), in particular its distal end region (16), has a planar top side (50), and **in that** the toothing (28; 30) is configured projecting at least partially beyond the top side (50),
wherein in particular the teeth (32; 34, 36, 38) are configured in such a way that each tooth flank (122) has a notch (134) in the second surface region (116) and **in that** the notch (134) is delimited by the first surface region (80) on the one hand and by the top side (50) on the other hand.

10. Method according to claim 9, **characterized in that** each first surface region (80) is configured such that it defines a first top side intersection line (124) with the top side (50), and **in that** each second surface region (116) is configured such that it defines a second top side intersection line (126) with the top side (50),
wherein in particular
a) each tooth flank (122) is configured such that the first top side intersection line (124) and the second top side intersection line (126) enclose an acute angle (128) opening in the proximal direction
and/or
b) the toothing (28; 30) is configured such that the second top side intersection lines (126) of adjacent teeth (32; 34, 36; 36, 38) define an acute angle (130) opened pointing in the distal direction
and/or
c) the toothing (28; 30) is configured such that the first surface region (80) and the second surface region (116) of a tooth flank (122) and the top side (50) come into contact at a point of intersection (132) of the first top side intersection line (124) and the second top side intersection line (126)
and/or
d) teeth (32; 34, 36, 38) are configured such that the first top side intersection line (124) extends in parallel or substantially in parallel to the longitudinal direction (80).

11. Method according to claim 9, **characterized in that** each first surface region (80) is configured such that together with a sloped face (160), which is inclined relative to the top side (50) by a slope angle (162), it defines a first sloped face intersection line (124') and **in that** each second surface region (116) is configured such that together with the sloped face (160) it defines a second sloped face intersection line (126').

12. Method according to claim 11, **characterized in that**
a) the slope angle (162) is configured having a value in a range of about 3° to about 30°, in particular having a value in a range of about 8° to about 13°, further in particular having a value of about 10°,
and/or
b) each first surface region (80) is configured such that the sloped face (160) and the top side (50) intersect in a sloped face top side intersection line (164), which extends transversely, in particular perpendicularly, to the longitudinal direction (48)
and/or
c) each tooth flank (122) is configured such that the first sloped face intersection line (124') and the second sloped face intersection line (126') of the same tooth flank (122) enclose an acute angle (128') opening in the proximal direction
and/or
d) the toothing (28; 30) is configured such that the second sloped face intersection lines (126') of adjacent teeth (32; 34, 36; 36, 38) of the toothing (28; 30) define an acute angle (130') opened pointing in the distal direction
and/or
e) the toothing (28; 30) is configured such that the first surface region (80) and the second surface region (116) of a tooth flank (122) and the sloped face (160) come into contact at a point of intersection (132') of the first sloped face intersection line (124') and the second sloped face intersection line (126')
and/or
f) teeth (32; 34, 36, 38) are configured such that the first sloped face intersection line (124') and the longitudinal direction (48) are inclined toward one another by the slope angle (162).

13. Method according to any one of claims 7 to 12, **characterized in that** the at least one first surface region (80) is formed with a first milling tool (68), in particular a cylindrical milling cutter (68),
wherein in particular a longitudinal axis (70; 70') of the cylindrical milling cutter (68) during milling
a) is oriented both perpendicularly to the top side (50) and perpendicularly to the longitudinal direction (48)
or
b) together with the longitudinal direction (48) defines a milling plane (158), which extends perpendicularly to the top side (50) and is inclined in the distal direction by a milling cutter longitudinal axis angle (156),
wherein in particular the milling cutter longitudinal axis angle (156) corresponds to the slope angle (162).

14. Method according to any one of claims 7 to 13, **characterized in that**
a) the at least one planar second surface region (116) is formed with a second milling tool (100), in particular a first conical milling cutter (100), wherein in particular a longitudinal axis (104) of the first conical milling cutter (100) during milling is inclined relative to the top side (50) by an angle of inclination (114) opened in the distal direction and together with the longitudinal direction (48) defines a plane extending perpendicularly to the top side (50),
and/or
b) each tooth (32; 34, 36, 38) is configured having at least one planar third surface region (88),
wherein in particular the at least one third surface region (88) is formed by cold forming or by milling, in particular with a CNC machine,
wherein further in particular the at least one third surface region (88) is formed with a third milling tool (90), in particular a second conical milling cutter (90), wherein in particular a longitudinal axis (92) of the second conical milling cutter (90) during milling is oriented both perpendicularly to the top side (50) and perpendicularly to the longitudinal direction (48).

15. Method in accordance with any one of claims 10 to 14, **characterized in that**
a) the toothing (28; 30) is configured such that each second surface region (116) defines a second surface region plane, **in that** the second surface region planes of adjacent teeth (32; 34, 36; 36, 38) define a common surface region intersection line, and **in that** the surface region intersection line together with the longitudinal direction (48) encloses an angle of inclination (114) opened in the distal direction,
wherein in particular the angle of inclination (114) is predetermined by an orientation of the longitudinal axis (92) of the second conical milling cutter (90) during milling relative to the top side (50) and wherein the longitudinal axis (92) is oriented perpendicularly to the surface region intersection line and together with the longitudinal direction (48) defines a plane extending perpendicularly to the top side (50), wherein in particular the angle of inclination (114) has a value in a range of about 30° to about 50°, in particular about 40°,
and/or
b) the instrument (10) is configured having two instrument arms (12, 14) such that a first toothing (28) is formed on a first instrument arm (12) of the two instrument arms (12, 14), the first toothing pointing in the direction toward a second instrument arm (14) of the two instrument arms (12, 14), **in that** a second toothing (30) is formed on the second instrument arm (14), the second toothing pointing in the direction toward the first instrument arm (12), and **in that** in a closing position of the instrument (10) in which distal ends (24, 26) of the two instrument arms (12, 14) are maximally proximate to one another, the first toothing (28) and the second toothing (30) interengage,
wherein in particular the second toothing (30) is configuring having one more tooth (34, 36, 38) than the first toothing (28).

## Revendications

1. Instrument médical (10) avec au moins un bras d'instrument (12 ; 14), une denture (28 ; 30) avec au moins deux dents (32 ; 34, 36, 38) étant formée à une extrémité distale (24 ; 26) du bras d'instrument (12 ; 14), lesquelles au moins deux dents font saillie transversalement de l'extrémité distale (24 ; 26), chacune des au moins deux dents (32 ; 34, 36, 38) présentant deux flancs de dent (122) s'écartant l'un de l'autre, chaque flanc de dent (122) présentant au moins une première zone de surface plane (80) et au moins une deuxième zone de surface plane (116), l'au moins une première zone de surface plane (80) et l'au moins une deuxième zone de surface plane (116) étant inclinées l'une par rapport à l'autre d'un angle de zone de surface (152) et les première et deuxième zones de surface (80, 116) étant réalisées par fraisage, en particulier avec une machine CNC.

2. Instrument médical selon la revendication 1, **caractérisé en ce qu'**une zone d'extrémité distale (16) de l'au moins un bras d'instrument (12, 14) définit une direction longitudinale (48) et **en ce que** la denture (28 ; 30) est réalisée en saillie transversale par rapport à la direction longitudinale (48).

3. Instrument médical selon l'une des revendications précédentes, **caractérisé en ce que** le bras d'instrument (12 ; 14), en particulier sa zone d'extrémité distale (16), présente une face supérieure plane (50) et **en ce que** la denture (28 ; 30) fait saillie au moins partiellement au-dessus de la face supérieure (50),
dans lequel, en particulier, chaque flanc de dent (122) présente une entaille (134) de la deuxième zone de surface (116) et dans lequel l'entaille (134) est délimitée d'une part par la première zone de surface (80) et d'autre part par la face supérieure (50).

4. Instrument médical selon la revendication 3, **caractérisé en ce que** chaque première zone de surface (80) définit avec la face supérieure (50) une première ligne de coupe de face supérieure (124) et **en ce que** chaque deuxième zone de surface (116) définit avec la face supérieure (50) une deuxième ligne de coupe de face supérieure (126),
dans lequel, en particulier,
a) la première ligne de coupe de face supérieure (124) et la deuxième ligne de coupe de face supérieure (126) du même flanc de dent (122) forment un angle aigu (128) s'ouvrant dans la direction proximale
et/ou
b) les deuxièmes lignes de coupe de face supérieure (126) de dents voisines (32 ; 34, 36 ; 36, 38) de la denture (28 ; 30) définissent un angle aigu (130) s'ouvrant dans la direction distale
et/ou
c) la première zone de surface (80) et la deuxième zone de surface (116) d'un flanc de dent (122) et la face supérieure (50) se touchent en un point de coupe (132) de la première ligne de coupe de face supérieure (124) et de la deuxième ligne de coupe de face supérieure (126)
et/ou
d) la première ligne de coupe de face supérieure (124) est parallèle ou sensiblement parallèle à la direction longitudinale (48).

5. Instrument médical selon la revendication 4, **caractérisé en ce que** chaque première zone de surface (80) ayant une surface inclinée (160) qui est inclinée d'un angle d'inclinaison (162) par rapport à la face supérieure (50) définit une première ligne de coupe de surface inclinée (124') et **en ce que** chaque deuxième zone de surface (116) avec la surface inclinée (160) définit une deuxième ligne de coupe de surface inclinée (126'),
dans lequel, en particulier,
a) l'angle d'inclinaison (162) présente une valeur dans une plage d'environ 3° à environ 30°, en particulier dans une plage d'environ 8° à environ 13°, plus particulièrement une valeur d'environ 10°,
et/ou
b) la surface inclinée (160) et la face supérieure (50) se coupent en une ligne de coupe de surfaces supérieures inclinées (164) qui est transversale, en particulier perpendiculaire, à la direction longitudinale
et/ou
c) la première ligne de coupe de surfaces inclinées (124') et la deuxième ligne de coupe de surfaces inclinées (126') du même flanc de dent (122) forment un angle aigu (128') s'ouvrant dans la direction proximale
et/ou
d) les deuxièmes lignes de coupe de surfaces inclinées (126') de dents adjacentes (32 ; 34, 36 ; 36, 38) de la denture (28 ; 30) définissent un angle aigu (130') orienté dans la direction distale
et/ou
e) la première zone de surface (80) et la deuxième zone de surface (116) d'un flanc de dent (122) et la surface inclinée (160) se touchent en un point de coupe (132') de la première ligne de coupe de surfaces inclinées (124') et de la deuxième ligne de coupe de surfaces inclinées (126')
et/ou
f) la première ligne de coupe de surfaces inclinées (124') et la direction longitudinale (48) sont inclinées l'une par rapport à l'autre autour de l'angle d'inclinaison (162).

6. Instrument médical selon l'une des revendications précédentes,
**caractérisé en ce que** l'instrument (10)
a) comprend deux bras d'instrument (12, 14), **en ce que**, sur un premier bras d'instrument (12) des deux bras d'instrument (12, 14), est formée une première denture (28) laquelle est orientée en direction d'un deuxième bras d'instrument (14) des deux bras d'instrument (12, 14), **en ce qu'**une deuxième denture (30) est formée sur le deuxième bras d'instrument (14), laquelle est orientée vers le premier bras d'instrument (12) et **en ce que**, dans une position fermée de l'instrument (10) dans laquelle les extrémités distales (24, 26) des deux bras d'instrument (12, 14) sont rapprochées au maximum l'une de l'autre, la première denture (28) et la deuxième denture (30) s'imbriquent l'une dans l'autre, la deuxième denture (30) comprenant une dent (34, 36, 38) de plus que la première denture (28),
et/ou
b) est réalisé sous la forme d'une pincette (154).

7. Procédé de fabrication d'un instrument médical (10), qui comprend au moins un bras d'instrument (12 ; 14), une denture (28 ; 30) avec au moins deux dents (32 ; 34, 36, 38) étant formée à une extrémité distale (24 ; 26) du bras d'instrument (12 ; 14), lesquelles au moins deux dents font saillie transversalement de l'extrémité distale (24 ; 26), chacune des au moins deux dents (32 ; 34, 36, 38) présentant deux flancs de dent (122) s'écartant l'un de l'autre, chaque flanc de dent (122) étant formé avec au moins une première zone de surface plane (80) et au moins une deuxième zone de surface plane (116), l'au moins une première zone de surface plane (80) et l'au moins une deuxième zone de surface plane (116) étant inclinées l'une par rapport à l'autre d'un angle de zone de surface (152) et les première et deuxième zones de surface (80, 116) étant formées par fraisage, en particulier avec une machine CNC.

8. Procédé selon la revendication 7, **caractérisé en ce qu'**une zone d'extrémité distale (16) de l'au moins un bras d'instrument (12 ; 14) définit une direction longitudinale (48) et **en ce que** la denture (28 ; 30) est réalisée en saillie transversale par rapport à la direction longitudinale (48).

9. Procédé selon la revendication 7 ou 8, **caractérisé en ce que** le bras d'instrument (12 ; 14), en particulier sa zone d'extrémité distale (16), présente une face supérieure (50) plane et **en ce que** la denture (28 ; 30) est réalisée au moins partiellement en saillie sur la face supérieure (50),
dans lequel, en particulier, les dents (32 ; 34, 36, 38) sont réalisées de telle sorte que chaque flanc de dent (122) présente une entaille (134) de la deuxième zone de surface (116) et dans lequel l'entaille (134) est limitée d'une part par la première zone de surface (80) et d'autre part par la face supérieure (50).

10. Procédé selon la revendication 9, **caractérisé en ce que** chaque première zone de surface (80) est formée de manière à définir avec la face supérieure (50) une première ligne de coupe de face supérieure (124), et **en ce que** chaque deuxième zone de surface (116) est formée de manière à définir avec la face supérieure (50) une deuxième ligne de coupe de face supérieure (126),
dans lequel, en particulier,
a) chaque flanc de dent (122) est formé de telle sorte que la première ligne de coupe de face supérieure (124) et la deuxième ligne de coupe de face supérieure (126) forment un angle aigu (128) s'ouvrant dans la direction proximale
et/ou
b) la denture (28 ; 30) est formée de telle sorte que les deuxièmes lignes de coupe de face supérieure (126) de dents voisines (32 ; 34, 36 ; 36, 38) définissent un angle aigu (130) orienté dans la direction distale
et/ou
c) la denture (28 ; 30) est réalisée de telle sorte qu'en un point de coupe (132) de la première ligne de coupe de face supérieure (124) et de la deuxième ligne de coupe de face supérieure (126), la première zone de surface (80) et la deuxième zone de surface (116) d'un flanc de dent (122) et la face supérieure (50) se touchent
et/ou
d) des dents (32 ; 34, 36, 38) sont formées de telle sorte que la première ligne de coupe de face supérieure (124) est parallèle ou sensiblement parallèle à la direction longitudinale (80).

11. Procédé selon la revendication 9, **caractérisé en ce que** chaque première zone de surface (80) est formée de manière à définir une première ligne de coupe de surfaces inclinées (124') avec une surface inclinée (160) qui est inclinée d'un angle d'inclinaison (162) par rapport à la face supérieure (50), et **en ce que** chaque deuxième zone de surface (116) est formée de manière à définir une deuxième ligne de coupe de surfaces inclinées (126') avec la surface inclinée (160).

12. Procédé selon la revendication 11, **caractérisé en ce que**
a) l'angle d'inclinaison (162) est formé avec une valeur se situant dans une plage d'environ 3° à environ 30°, en particulier avec une valeur se situant dans une plage d'environ 8° à environ 13°, plus particulièrement avec une valeur d'environ 10°,
et/ou
b) chaque première zone de surface (80) est formée de telle sorte que la surface inclinée (160) et la face supérieure (50) se coupent selon une ligne de coupe de surfaces supérieures inclinées (164) qui s'étend transversalement, en particulier perpendiculairement, à la direction longitudinale (48)
et/ou
c) chaque flanc de dent (122) est formé de telle sorte que la première ligne de coupe de surfaces inclinées (124') et la deuxième ligne de coupe de surfaces inclinées (126') du même flanc de dent (122) forment un angle aigu (128') s'ouvrant dans la direction proximale
et/ou
d) la denture (28 ; 30) est réalisée de telle sorte que les deuxièmes lignes de coupe de surfaces inclinées (126') de dents voisines (32 ; 34, 36 ; 36, 38) de la denture (28 ; 30) définissent un angle aigu (130') ouvert dans la direction distale
et/ou
e) la denture (28 ; 30) est réalisée de telle sorte qu'en un point de coupe (132') de la première ligne de coupe de surfaces inclinées (124') et la deuxième ligne de coupe de surfaces inclinées (126'), la première zone de surface (80) et la deuxième zone de surface (116) d'un flanc de dent (122) et la surface inclinée (160) se touchent
et/ou
f) des dents (32 ; 34, 36, 38) sont formées de telle sorte que la première ligne de coupe de surfaces inclinées (124') et la direction longitudinale (48) sont inclinées l'une par rapport à l'autre de l'angle d'inclinaison (162).

13. Procédé selon l'une des revendications 7 à 12, **caractérisé en ce que** l'au moins une première zone de surface (80) est formée avec un premier outil de fraisage (68), en particulier une fraise cylindrique (68), dans lequel, en particulier, un axe longitudinal (70 ; 70') de la fraise cylindrique (68), lors du fraisage,
a) est orienté à la fois perpendiculairement à la face supérieure (50) et perpendiculairement à la direction longitudinale (48)
ou
b) définit avec la direction longitudinale (48) un plan de fraisage (158) qui s'étend perpendiculairement à la face supérieure (50) et est incliné dans la direction distale d'un angle d'axe longitudinal de fraisage (156),
dans lequel, en particulier, l'angle d'axe longitudinal de fraisage (156) correspond à l'angle d'inclinaison (162).

14. Procédé selon l'une quelconque des revendications 7 à 13, **caractérisé en ce que**
a) l'au moins une deuxième zone de surface plane (116) est formée avec un deuxième outil de fraisage (100), en particulier une première fraise conique (100), un axe longitudinal (104) de la première fraise conique (100) étant en particulier incliné lors du fraisage par rapport à la face supérieure (50) d'un angle d'inclinaison (114) ouvert en direction distale et définissant, avec la direction longitudinale (48), un plan s'étendant perpendiculairement à la face supérieure (50),
et/ou
b) chaque dent (32 ; 34, 36, 38) étant formée avec au moins une troisième zone de surface plane (88),
dans lequel, en particulier, l'au moins une troisième zone de surface (88) est formée par formage à froid ou par fraisage, en particulier avec une machine CNC,
dans lequel, en outre en particulier, l'au moins une troisième zone de surface (88) est formée avec un troisième outil de fraisage (90), en particulier une deuxième fraise conique (90), dans lequel en particulier un axe longitudinal (92) de la deuxième fraise conique (90) est orienté lors du fraisage aussi bien perpendiculairement à la face supérieure (50) que perpendiculairement à la direction longitudinale (48).

15. Procédé selon l'une quelconque des revendications 10 à 14, **caractérisé en ce que**
a) la denture (28 ; 30) est réalisée de telle sorte que chaque deuxième zone de surface (116) définit un deuxième plan de zone de surface, **en ce que** les deuxièmes plans de zone de surface de dents voisines (32 ; 34, 36 ; 36, 38) définissent une ligne de coupe de zones de surfaces commune et **en ce que** la ligne de coupe de zones de surfaces avec la direction longitudinale (48) inclut un angle d'inclinaison (114) ouvert dans la direction distale, dans lequel, en particulier, l'angle d'inclinaison (114) est prédéfini par une orientation de l'axe longitudinal (92) de la deuxième fraise conique (90) lors du fraisage par rapport à la face supérieure (50) et dans lequel l'axe longitudinal (92) est orienté perpendiculairement à la ligne de coupe de la zone de surface et définit avec la direction longitudinale (48) un plan s'étendant perpendiculairement à la face supérieure (50), dans lequel l'angle d'inclinaison (114) présente une valeur située dans une plage d'environ 30° à environ 50°, en particulier d'environ 40°,
et/ou
b) l'instrument (10) est réalisé avec deux bras d'instrument (12, 14) de telle sorte qu'une première denture (28) est réalisée sur un premier bras d'instrument (12) des deux bras d'instrument (12, 14), laquelle est orientée en direction d'un deuxième bras d'instrument (14) des deux bras d'instrument (12, 14), **en ce qu'**une deuxième denture (30) est formée sur le deuxième bras d'instrument (14), qui est orientée vers le premier bras d'instrument (12) et **en ce que**, dans une position de fermeture de l'instrument (10), dans laquelle les extrémités distales (24, 26) des deux bras d'instrument (12, 14) se rapprochent au maximum l'une de l'autre, la première denture (28) et la deuxième denture (30) s'imbriquent,
dans lequel, en particulier, la deuxième denture (30) est réalisée avec une dent (34, 36, 38) de plus que la première denture (28).
